# EUROPÄISCHE PATENTSCHRIFT

(11) **EP 2 665 717 B1**
(45) Veröffentlichungstag und Bekanntmachung des Hinweises auf die Patenterteilung: **24.06.2015**
(21) Anmeldenummer: 11804966.7
(22) Anmeldetag: 27.12.2011
(51) Int. Cl.: C07D 311/22, A61K 8/49, A61Q 17/04, A61Q 19/04

(54) **7-ACYLOXY-CHROMEN-4-ON-DERIVATE UND IHRE VERWENDUNG ALS SELBSTBRÄUNUNGSSUBSTANZEN**
7-ACYLOXY-CHROMEN-4-ONE DERIVATIVES AND THEIR USE AS SELF-TANNING SUBSTANCES
DÉRIVÉS DE 7-ACYLOXY-CHROMÈNE-4-ONE ET LEUR UTILISATION EN TANT QUE SUBSTANCES AUTO-BRONZANTES

(30) Priorität: 21.01.2011 DE 102011009112
(43) Veröffentlichungstag der Anmeldung: 27.11.2013
(73) Patentinhaber: Merck Patent GmbH, 64293 Darmstadt (DE)
(72) Erfinder: CAROLA, Christophe, 64625 Bensheim (DE); SCHEURICH, René Peter, 64846 Gross-Zimmern (DE)
(86) Internationale Anmeldenummer: PCT/EP2011/006574
(87) Internationale Veröffentlichungsnummer: WO 2012/097857

(56) Entgegenhaltungen:
- WO-A1-2007/087956
- DATABASE WPI Week 199739 Thomson Scientific, London, GB; AN 1997-420525 XP002668584, -& JP 9 188608 A (KAO CORP) 22. Juli 1997 (1997-07-22) in der Anmeldung erwähnt

## Beschreibung

Die vorliegende Erfindung betrifft Chromen-4-on-Derivate der Formel I, ihre Verwendung als Selbstbräunungssubstanz oder zur Steigerung der Melaninsynthese, Verbesserung des Melanintransports und/oder Verbesserung der Verteilung von Melanin in suprabasalen Schichten, sowie Zubereitungen enthaltend diese Chromen-4-on-Derivate.

Der Trend weg von der vornehmen Blässe hin zur "gesunden, sportlich braunen Haut" ist seit Jahren ungebrochen. Um einen gebräunten Teint zu erzielen, setzen die Menschen ihre Haut der Sonnenstrahlung aus, da diese eine Pigmentierung durch eine Melaninbildung hervorruft. Die UV-Strahlung des Sonnenlichtes hat jedoch auch eine schädigende Wirkung auf die Haut. Neben der akuten Schädigung (Sonnenbrand) treten Langzeitschäden bei übermäßiger Bestrahlung mit Licht aus dem UVB-Bereich (Wellenlänge 280-320 nm) auf, wie beispielsweise ein erhöhtes Risiko, an Hautkrebs zu erkranken. Die übermäßige Einwirkung der UVB-und UVA-Strahlung (Wellenlänge: 320-400 nm) erzeugt hochreaktive Radikalspezies, die sich auch nach Beendigung der Bestrahlung weiter vermehren und als deren Folge es zu Faltenbildung und Hautalterung kommt.

Den natürlichen Schutz vor den negativen Folgen der Sonnenstrahlung bietet die Bräunung (Pigmentierung) der Haut. Die Epidermis enthält in ihrer untersten Schicht, der Basalschicht, neben den Basalzellen einzelne pigmentbildende Zellen, die Melanocyten. Durch UV-Licht wird in diesen Zellen die Produktion von Melanin angeregt, das in die Kerantinocyten (Homzellen) transportiert und dort als braune Hautfarbe sichtbar wird. Melanin schützt die Zellkerne vor weiterer Bestrahlung und den davon verursachten negativen Auswirkungen auf die Zell-DNA.

Je nach chemischer Zusammensetzung der biochemisch gebildeten Pigmente wird zwischen dem bräunlich-schwarzen Eumelanin und dem rötlich-gelben Pheomelanin unterschieden. Der beobachtete Hautfarbton wird vom Verhältnis dieser beiden Melaninarten bestimmt. Diese von der Aminosäure Tyrosin ausgehende Pigmentbildung wird überwiegend durch UVB-Strahlung initiiert und als "indirekte Pigmentierung" bezeichnet. Ihre Entwicklung läuft über mehrere Tage; die so erhaltene Sonnenbräune besteht über einige Wochen. Bei der "Direkt-Pigmentierung", die mit der Sonnenbestrahlung einsetzt, werden vorwiegend farblose Melanin-Vorstufen durch UVA-Strahlung zu dunkel gefärbtem Melanin oxidiert. Da diese Oxidierung reversibel ist, führt sie zu einer nur kurz anhaltenden Hautbräunung.

Eine künstliche Bräunung der Haut lässt sich äußerlich mit Hilfe von Schminke und oral durch Einnahme von Carotinoiden erzeugen.

Weitaus beliebter jedoch ist die künstliche Bräunung der Haut, welche sich durch Auftragen von sogenannten Selbstbräunern erzielen lässt. Diese Verbindungen weisen als chemisches Strukturmerkmal Keto- bzw. Aldehydgruppen in Nachbarschaft zu Alkohotfunktionen auf und gehören überwiegend zur Substanzklasse der Zucker. Besonders häufig eingesetzte Selbstbräunungssubstanzen sind 1,3-Dihydroxyaceton (DHA), das in einer Menge von 700t/a verwendet wird, und Erythrulose.

Selbstbräuner können mit den Proteinen und Aminosäuren der Hornschicht der Haut im Sinne einer Maillard-Reaktion oder über eine Michael Addition umgesetzt werden, wobei über einen noch nicht vollständig aufgeklärten Reaktionsweg Polymerisate entstehen, die der Haut einen bräunlichen Farbton verleihen. Diese Reaktion ist nach etwa 4 bis 6 Stunden abgeschlossen. Die so erzielte Bräune ist nicht abwaschbar und wird erst mit der normalen Hautabschuppung entfernt.

Diese Farbprodukte besitzen allerdings selbst keine UV-absorbierenden Eigenschaften, sodass bei Sonnenexposition ein zusätzlicher Sonnenschutz (Bekleidung, Hut, UV-Filter) erforderlich wird. Im Gegensatz zu "sonnengebräunter" Haut ist die so gebräunte Haut nicht gegen Sonnenbrand geschützt.

Es besteht daher weiterhin ein Bedarf nach dermatologisch verträglichen hautfärbenden Substanzen, die sich zum Einsatz in kosmetischen und/oder dermatologischen Zubereitungen oder Medizinprodukten eignen und die die natürliche Bräunung der Haut durch Steigerung der Melaninsynthese verstärken und gleichzeitig einen besseren Hauteigenschutz bzw. Sonnenschutz, insbesondere gegen UVB-Strahlung, ermöglichen.

Die der vorliegenden Erfindung zugrunde liegende Aufgabe bestand daher in der Bereitstellung von neuen Selbstbräunungssubstanzen mit verbesserten Eigenschaften.

Überraschenderweise wurde nun festgestellt, dass sich bestimmte Chromen-4-on-Derivate (Chromon-Derivate) als Selbstbräunungssubstanzen eignen.

Im Sinne der Erfindung wird der Begriff Selbstbräunungswirkstoff synonym zu Selbstbräunungssubstanz oder Selbstbräunersubstanz verwendet.

Ähnliche Anwendungen strukturell verwandter Verbindungen sind aus der Literatur bekannt:
Zubereitungen zur topischen Anwendung, die Chromon-Derivate, wie z.B. Chromon, 7-Hydroxychromon, 7-Methoxychromon, 5,7-Dihydroxy-2-methyl-chromon, 3-Methyl -2-butenyloxy-chromon, 3-Acetyl-5,7-dihydroxy-2-methyl-chromon, 5-hydroxychromon, n-Pentyl-7-methoxychromon-2-carboxylat, n-Undecyl-5-rnethoxychromon-2-carboxylat, 5-Hydroxy-7-methoxy-2-methyl-chromon, 7-Methoxy-chromon-2-carbonsäure, n-Pentyl-chromon-2-carbonsäure, 5-methoxychromon und Chromon-2-carbonsäure, enthalten, sind aus der japanischen Patentanmeldung JP 05/301813 bekannt. Die Chromon-Derivate wirken als hautverträgliche Tyrosinase-Inhibitoren, welche die Hyperpigmentierung der Haut verringern.

Aus der Japanischen Patentanmeldung JP 09/188608 ist die Verwendung von substituierten Chromon-Derivaten, wie insbesondere 5,7-Dihydroxychromone, 7-Methoxychromone, 5-Hydroxy-7-methoxy-2-methyl-chromon und 5-Hydroxy-2-methyl-chromon, als Wirkstoff gegen graue Haare bekannt. Die Wirkung wird dabei auf die Aktivierung der Farbpigmentbildenden Zellen und die Steigerung der Melanogenese zurückgeführt. Die in dieser japanischen Anmeldung offenbarte generische Formel umfasst auch Chromon-Derivate, die an der Position 7 kurze Acyloxygruppen mit 1 bis 5 C-Atomen aufweisen. Im Rahmen der vorliegenden Erfindung konnte jedoch gezeigt werden, dass 5-Hydroxy-7-Acetyl-2-methyl-chromon im *ex vivo*-Experiment keine Bräunungsaktivität aufweist.

WO 2007/087956 A1 beschreibt Chromon-Derivate, die an Position 7 eine Alkoxy- oder Hydroxygruppe aufweisen und eine Bräunungsaktivität besitzen.

Die Anmeldung US 2010/0158829 A1 offenbart die Verwendung von verschiedenen Chromon-Derivaten zur Farbveränderung der Haut, insbesondere zur Hautaufhellung durch Hemmung des Melanintransfers von den Melanosomen zu den Keratinocyten.

Ein erster Gegenstand der vorliegenden Erfindung sind daher Verbindungen der Formel I 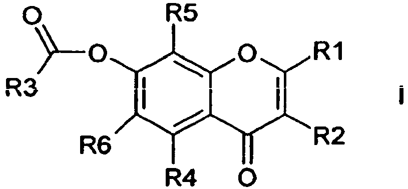 wobei
R1 und R2 unabhängig voneinander stehen für
   -H,
   -OH,
   -geradkettiges oder verzweigtes O-(C₁- bis C₆-Alkyl) oder
   -geradkettiges oder verzweigtes C₁- bis C₆-Alkyl,
   wobei R1 und/oder R2 durch eine oder mehrere OH-Gruppen substituiert sein können und/oder wobei ein oder mehrere nicht benachbarte CH₂-Gruppen durch O ersetzt sein können;
R3 steht für
   -geradkettige oder verzweigte C₆- bis C₂₀-Alkylgruppe,
   -geradkettige oder verzweigte C₂- bis C₂₀-Alkenylgruppe,
   -geradkettige oder verzweigte C₂- bis C₂₀-Alkinylgruppe,
   -eine Cycloalkyl- oder Cycloalkenylgruppe mit 3 bis 6 C-Atomen, oder
   -einen Rest der Formel III 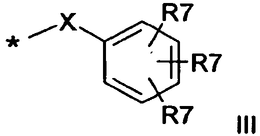 worin X für ein geradkettiges oder verzweigtes C₁- bis C₆-Alkylen oder geradkettiges oder verzweigtes C₂- bis C₆-Alkenylen steht und die Reste R7 unabhängig voneinander ausgewählt sind aus H, OH, geradkettiges oder verzweigtes C₁- bis C₆-Alkyl oder geradkettiges
   oder verzweigtes O-(C₁- bis C₆-Alkyl),
R4 für H, OH oder geradkettiges oder verzweigtes O-(C₁- bis C₂₀-Alkyl) steht,
R5 und R6 unabhängig voneinander stehen für
   -H,
   -OH,
   -geradkettige oder verzweigte C₁- bis C₂₀-Alkylgruppe,
   -geradkettige oder verzweigte C₂- bis C₂₀-Alkenylgruppe,
   -geradkettige oder verzweigte C₁- bis C₂₀-Hydroxyalkylgruppe, wobei die Hydroxygruppe an ein primäres oder sekundäres Kohlenstoffatom der Kette gebunden sein kann und/oder ein oder mehrere nicht benachbarte CH₂-Gruppen durch O ersetzt sein können, oder
   -geradkettige oder verzweigte O-(C₁- bis C₂₀-Alkyl)-Gruppe.

Grundsätzlich sind im Sinne der vorliegenden Erfindung von der Bezeichnung "Verbindung nach Formel I" auch die Salze der jeweiligen Verbindungen nach Formel I umfasst. Zu den bevorzugten Salzen gehören dabei insbesondere Alkali- und Erdalkalimetallsalze, Zink- sowie Ammonium-Salze, insbesondere jedoch Natrium- und Kalium-Salze.

R1 steht bevorzugt für H, OH, OCH₃, CH₃, CH₂OH oder CH₂OCH₃. Besonders bevorzugt steht R1 für CH₂OCH₃, CH₂OH oder CH₃, ganz besonders bevorzugt für CH₃.

R2 steht in Formel I bevorzugt für H, OH, OCH₃, CH₃, CH₂OH oder CH₂OCH₃. Besonders bevorzugt steht R2 für H, OH oder OCH₃, ganz besonders bevorzugt für H.

R3 steht bevorzugt für
- geradkettige oder verzweigte C₆- bis C₂₀-Alkylgruppe,
- geradkettige oder verzweigte C₂- bis C₂₀-Alkenylgruppe, oder
- einen Rest der Formel III 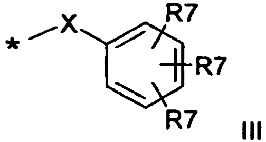 worin X für ein geradkettiges oder verzweigtes C₁- bis C₆-Alkylen oder geradkettiges oder verzweigtes C₂- bis C₆-Alkenylen steht und die Reste R7 unabhängig voneinander ausgewählt sind aus H, OH, geradkettiges oder verzweigtes C₁- bis C₆-Alkyl oder geradkettiges oder verzweigtes O-(C₁- bis C₆-Alkyl).

Im Rest der Formel III steht X für bevorzugt für -CH₂-, -CH₂CH₂-, -CH=CH-, -CH₂-CH=CH- oder -CH=CH-CH₂-, besonders bevorzugt für -CH₂CH₂-oder -CH=CH-. R7

Ganz besonders bevorzugt ist R3 ausgewählt aus der Gruppe umfassend 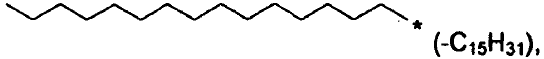 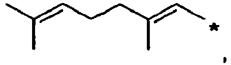 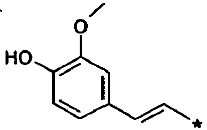 und

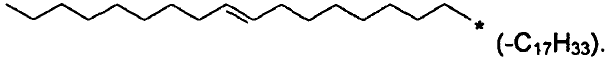

R4 steht bevorzugt für H, OH oder eine geradkettige oder verzweigte C₁-bis C₆-Alkylgruppe, besonders bevorzugt für OH oder OCH₃, ganz besonders bevorzugt für OH.

R5 steht bevorzugt für H oder OH, besonders bevorzugt für H.

R6 steht bevorzugt für H oder OH, besonders bevorzugt für H.

Die Reste R7 sind bevorzugt unabhängig voneinander ausgewählt aus H, OH und OCH₃, besonders bevorzugt steht ein Rest R7 für H, ein Rest R7 für OH und ein Rest R7 für OCH₃.

In einer bevorzugten Ausführungsform der vorliegenden Erfindung steht die Verbindung der Formel I für eine Verbindung der Formel II 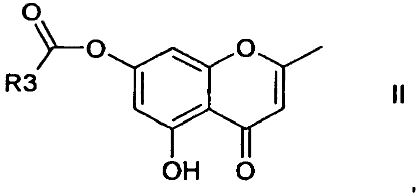 worin R3 wie zuvor beschrieben definiert ist.

Besonders bevorzugt ist die Verbindung der Formel I ausgewählt aus den Verbindungen der Formel Ia, Ib, Ic und Id

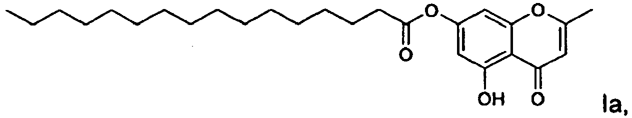

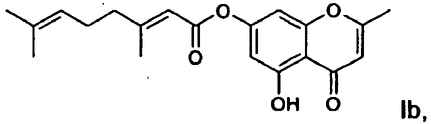

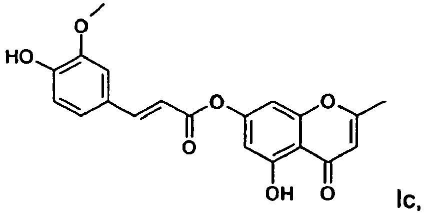

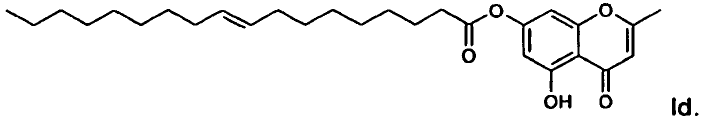

Ganz besonders bevorzugt sind Verbindungen der Formel la.

Die erfindungsgemäßen Verbindungen der Formel I, wie zuvor definiert, können durch Cyclisierung eines entsprechend substituierten o-Hydroxyacetophenons mit einem Anhydrid oder mit einem Acylchlorid unter basischen Bedingungen erhalten werden. Anschließend können die Acylgruppen, d.h. zum einen O-Acylgruppen, die durch Reaktion der freien Hydroxygruppe mit dem Anhydrid oder Acylchlorid entstanden sind, zum anderen C-Acylgruppen in Position 3 des Chromonsystems, entfernt werden. Dabei kann die Umsetzung analog zu Kelly, T; Kim M.H.; J. Org. Chem. 1992, 57, 1593-97 erfolgen.
Durch übliche Umsetzungen an dem Ringsystem oder Derivatisierung der funktionellen Gruppen können weitere Derivate gemäß Formel I erhalten werden. Die dazu notwendigen Reaktionsbedingung für solche Reaktionen, wie beispielsweise Oxidationen, Reduktionen, Umesterungen, Veretherungen, findet ein Fachmann für derartige Synthesen problemlos in der allgemein zugänglichen Literatur zu organischen Reaktionen.
Die Esterfunktion in Position 7 des Chromonsystems kann dabei durch eine einfache Veresterungsreaktion eingeführt werden.

Ein weiterer Gegenstand der vorliegenden Erfindung ist daher auch ein Verfahren zur Herstellung einer Verbindung der Formel I wie zuvor definiert, dadurch gekennzeichnet, dass ein o-Hydroxyacetophenon der Formel IV 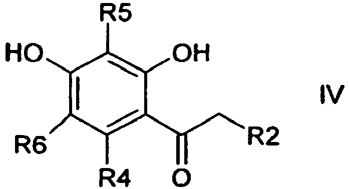 wobei die Substituenten R2 bis R6 eine der zuvor beschriebenen oder als bevorzugt beschriebenen Bedeutungen haben mit Acetylchlorid unter basischen Bedingungen cyclisiert wird und das Produkt anschließend in einer Veresterungsreaktion mit einer Verbindung der Formel R3-COCl umgesetzt wird.

Bevorzugt findet die Cyclisierung in einem Lösungsmittel statt, ausgewählt aus der Gruppe Tetrahydrofuran, Diethylether, Diisopropylether, Methyl-terbutylether oder 1,4-Dioxan.

Die Cyclisierung erfolgt vorzugsweise unter Inertgasbedingungen. Vorteilhaft wird K₂CO₃ oder ein anderes Reagenz aus der Gruppe KO-tBu (Kaliumtertbutoxid), LiOH, KOH oder DBU in Pyridin bei Temperaturen zwischen -10°C und 35°C, bevorzugt bei Raumtemperatur, eingebracht. Die eigentliche Reaktionstemperatur liegt zwischen -10 und 175°C, bevorzugt zwischen 35 und 100°C. Besonders bevorzugt erfolgt die Umsetzung bei 50°C oder 66°C.

Im Sinne der vorliegenden Erfindung handelt es sich bei einer geradkettigen oder verzweigten Alkylgruppe mit 1 bis 6 C-Atomen beispielsweise um Methyl, Ethyl, Isopropyl, Propyl, Butyl, sek.-Butyl oder tert.-Butyl, Pentyl, Isopentyl, 1-, 2- oder 3-Methylbutyl, 1,1-, 1,2- oder 2,2-Dimethylpropyl, 1-Ethylpropyl, 1-Ethyl-1-methylpropyl, 1-Ethyl-2-methylpropyl, 1,1,2-oder 1,2,2-Trimethylpropyl, 1,1-, 1,2-, 1,3-, 2,2-, 2,3-oder 3,3-Dimethylbutyl, 1- oder 2-Ethylbutyl, 1-, 2-, 3- oder 4-Methylpentyl oder Hexyl.
Bei einem Alkylrest mit 1 bis 20 C-Atomen kann es sich neben den oben gelisteten Resten beispielsweise auch um Heptyl, 1-Ethyl-pentyl, Octyl, 1-Ethyl-hexyl, Nonyl, Decyl, Undecyl, Dodecyl, Tridecyl, Tetradecyl, Pentadecyl, Hexadecyl, Heptadecyl, Octadecyl, Nonadecyl oder Eicosyl handeln.
Unter einem Alkylrest mit 6 bis 20 C-Atomen werden die gleichen Reste wie beschrieben verstanden, wobei jedoch die Reste Methyl, Ethyl, Isopropyl, Propyl, Butyl, sek.-Butyl oder tert.-Butyl, Pentyl, Isopentyl, 1-, 2- oder 3-Methylbutyl, 1,1-, 1,2- oder 2,2-Dimethylpropyl und 1-Ethylpropyl aus der obigen Liste ausgeschlossen sind.

Bei einem C₁- bis C₆-Alkylenrest handelt es sich beispielsweise um Methylen, Ethylen, Propylen, Butylen, Pentylen oder Hexylen.

Erfindungsgemäß kann eine Alkenylgruppe eine oder mehrere Doppelbindungen enthalten. Eine verzweigte oder nicht verzweigte Alkenylgruppe mit 2 bis 20 C-Atomen ist beispielsweise Allyl, Vinyl, Propenyl, 2- oder 3-Butenyl, Isobutenyl, sek.-Butenyl, 2-Methyl-1- oder 2-Butenyl, 3-Methyl-1-butenyl, 1,3-Butadienyl, 2-Methyl-1,3-butadienyl, 2,3-Dimethyl-1,3-butadienyl, 1-, 2-, 3- oder 4-Pentenyl, iso-Pentenyl, Hexenyl, Heptenyl oder Octenyl, -C₉H₁₇, -C₁₀H₁₉ bis -C₂₀H₃₉.

Beispiele für einen C₂- bis C₆-Alkenylenrest sind Ethenylen, Propenylen, 2-oder 3-Butenylen, 1-, 2-, 3- oder 4-Pentenylen oder Hexenylen.

Ein Alkinylrest kann ein oder mehrere Dreifachbindungen enthalten. Beispiele für eine verzweigte oder nicht verzweigte Alkinylgruppe mit 2 bis 20 C-Atomen sind Ethinyl, 1- oder 2-Propinyl, 2- oder 3-Butinyl, ferner 4-Pentinyl, 3-Pentinyl, Hexinyl, Heptinyl, Octinyl, -C₉H₁₅, -C₁₀H₁₇ bis -C₂₀H₃₇.

Eine Cycloalkyl- oder Cycloalkenylgruppe enthält im Sinne der vorliegenden Erfindung 3 bis 6 C-Atome. Cycloalkenylgruppen enthalten eine oder zwei Doppelbindungen. Beispiele für Cylclalkyl- oder Cyclyalkenylgruppen mit 3 bis 6 C-Atomen sind Cyclopropyl, -butyl, -pentyl oder -hexyl, weiterhin auch Cyclopentenyl, -hexenyl oder -hexadienyl.

Die Verbindungen der Formel I können in Selbstbräunungsprodukten als Selbstbräunungssubstanz und/oder zur Steigerung der Melaninsynthese in der Haut, zur Verbesserung des Melanintransports und/oder zur Verbesserung der Verteilung von Melanin in suprabasalen Schichten und/oder zum Schutz der Haut vor schädigenden UV-Strahlen eingesetzt werden.

Ein weiterer Gegenstand der vorliegenden Erfindung ist daher die Verbindung der Formel I, wie zuvor beschrieben, zur Verwendung als Selbstbräunungssubstanz.

Ebenfalls Gegenstand der vorliegenden Erfindung ist die Verbindung der Formel I, wie zuvor beschrieben, zur Verwendung zur Steigerung der Melaninsynthese, Verbesserung des Melanintransports und/oder Verbesserung der Verteilung von Melanin in suprabasalen Schichten.

Verbindungen der Formel I steigern die Melaninsynthese und verbessern den Melanintransport von den Melanocyten zu den Keratinocyten. Dies wirkt sich auf die Farbe der Haut aus und bewirkt einen Bräunungseffekt.

Die Verbindungen der Formel I können neben der Bräunungswirkung auch eine antioxidante Wirkung haben und weisen eine gute Hautverträglichkeit auf. Zusätzlich sind bevorzugte der hier beschriebenen Verbindungen farblos oder nur schwach gefärbt und führen so nicht oder nur in geringer Weise zu Verfärbungen der Zubereitungen. Die bevorzugten Verbindungen weisen außerdem eine verbesserte Löslichkeit in kosmetischen Ölen auf.

Damit die Verbindungen der Formel I ihre positive Wirkung auf die Haut besonders gut entwickeln können, kann es bevorzugt sein die Verbindungen der Formel I, wie zuvor beschrieben, in tiefere Hautschichten eindringen zu lassen. Dazu stehen mehrere Möglichkeiten zur Verfügung. Zum einen können die Verbindungen der Formel I eine ausreichende Lipophilie aufweisen, um durch die äußere Hautschicht in epidermale Schichten vordringen zu können. Als weitere Möglichkeit können in der Zubereitung auch entsprechende Transportmittel, beispielsweise Liposomen, vorgesehen sein, die einen Transport der Verbindungen der Formel I durch die äußeren Hautschichten ermöglichen. Schließlich ist auch ein systemischer Transport der Verbindungen der Formel I denkbar. Die Zubereitung wird dann beispielsweise so gestaltet, dass sie für eine orale Gabe geeignet ist.

Die erfindungsgemäße Verwendung erfolgt bevorzugt nicht-therapeutisch.

Ein weiterer Gegenstand der vorliegenden Erfindung ist eine Zubereitung enthaltend mindestens eine Verbindung der Formel I oder deren bevorzugte Ausführungsformen, wie zuvor beschrieben definiert.

Bei den Zubereitungen handelt es sich dabei üblicherweise um topisch anwendbare Zubereitungen, beispielsweise kosmetische oder dermatologische Formulierungen oder Medizinprodukte. Die Zubereitungen enthalten in diesem Fall einen kosmetisch oder dermatologisch geeigneten Träger und je nach gewünschtem Eigenschaftsprofil optional weitere geeignete Inhaltsstoffe. Handelt es sich um pharmazeutische Zubereitungen, so enthalten die Zubereitungen in diesem Fall einen pharmazeutisch verträglichen Träger und optional weitere pharmazeutische Wirkstoffe.

Im Sinne der vorliegenden Erfindung wird neben dem Begriff Zubereitung gleichbedeutend auch der Begriff Mittel oder Formulierung verwendet.

Topisch anwendbar bedeutet im Sinne der Erfindung, dass die Zubereitung äußerlich und örtlich angewendet wird, d.h. dass die Zubereitung geeignet sein muss, um beispielsweise auf die Haut aufgetragen werden zu können.

Die Zubereitungen können die genannten notwendigen oder optionalen Bestandteile umfassen oder enthalten, daraus im wesentlichen oder daraus bestehen. Alle Verbindungen oder Komponenten, die in den Zubereitungen verwendet werden können, sind entweder bekannt und käuflich erwerbbar oder können nach bekannten Verfahren synthetisiert werden.

Bevorzugt handelt es sich um eine kosmetische oder pharmazeutische Zubereitung; besonders bevorzugt handelt es sich um eine kosmetische Zubereitung.

Die mindestens eine Verbindung der Formel I wird in den erfindungsgemäßen Zubereitungen typischerweise in Mengen von 0,01 bis 10 Gew.-%, bevorzugt in Mengen von 0,05 bis 10 Gew.-%, besonders bevorzugt in Mengen von 0,1 Gew.-% bis 5 Gew.-% und ganz besonders bevorzugt in Mengen von 0,5 bis 2 Gew.-%, bezogen auf die Gesamtmenge der Zubereitung, eingesetzt. Dabei bereitet es dem Fachmann keinerlei Schwierigkeiten die Mengen abhängig von der beabsichtigten Wirkung der Zubereitung entsprechend auszuwählen.

Ferner können die erfindungsgemäßen Zubereitungen mindestens eine weitere Selbstbräunungssubstanz als weiteren Inhaltsstoff enthalten. Dabei kann es sich sowohl um einen Selbstbräuner handeln, der mit den Aminosäuren der Haut im Sinne einer Maillard-Reaktion oder über eine Michael-Addition reagiert, als auch um einen sogenannten Melanogenese-Promotor oder Propigmentierungswirkstoff, der die natürliche Bräunung der Haut fördert.
Als vorteilhafte Selbstbräunungssubstanzen können unter anderem eingesetzt werden: 1,3-Dihydroxyaceton, Glycerolaldehyd, Hydroxymethylglyoxal, γ-Dialdehyd, Erythrulose, 6-Aldo-D-Fructose, Ninhydrin, 5-Hydroxy-1,4-naphtochinon (Juglon) oder 2-Hydroxy-1,4-naphtochinon (Lawson). Ganz besonders bevorzugt ist 1,3-Dihydroxyaceton, Erythrulose oder deren Kombination. Propigmentierungsstoffe können prinzipiell alle dem Fachmann bekannten Wirkstoffe sein. Beispiele hierfür sind Glycyrrhetinsäure, Melanocytenstimulierendes Hormon (alpha-MSH), Peptidanaloga, Thymidin-Dinucleotide, L-Tyrosin und dessen Ester oder bicyclische Monoterpendiole (beschrieben in Brown et al., Photochemistry and Photobiology B: Biology 63 (2001) 148-161).

Bevorzugt ist die mindestens eine weitere Selbstbräunungssubstanz in der Zubereitung in einer Menge von 0,01 bis 20 Gew.-%, besonders bevorzugt in einer Menge von 0,5 bis 15 Gew.-% und ganz besonders bevorzugt in einer Menge von 1 bis 8 Gew.-%, bezogen auf die Gesamtmenge der Zubereitung, enthalten.

Zubereitungen mit Selbstbräunereigenschaften, insbesondere solche, die Dihydroxyaceton enthalten, neigen bei der Anwendung auf der menschlichen Haut zu Fehlgerüchen, die vermutlich durch Abbauprodukte des Dihydroxyacetons selbst oder durch Produkte von Nebenreaktionen verursacht werden und die von den Anwendem teilweise als unangenehm empfunden werden. Es hat sich gezeigt, dass diese Fehlgerüche bei Verwendung von Formaldehydfängern und/oder Flavonoiden vermieden werden. Daher kann die erfindungsgemäße Zubereitung auch Formaldehydfänger sowie gegebenenfalls Flavonoide zur Verbesserung des Geruches enthalten.

Die erfindungsgemäße Zubereitung, welche eine Selbstbräunungssubstanz und ein Chromon der Formel I kombiniert, hat gegenüber einem Selbstbräunungsprodukt ohne Chromon-Zugabe folgende Vorteile:
- Beschleunigung der Bräunungs-Reaktion,
- Verlängerung der Bräunungs-Reaktion aufgrund der indirekten Bräunungs-Reaktion (UV-freie Bräunungsverlängerung),
- Verstärkung der Bräunungs-Reaktion,
- Vermeidung von ungleichmäßiger Bräunung durch ungeschicktes Auftragen,
- die erzielte Bräunung kommt der natürlichen Bräunung nahe,
- Verbesserung des Schutzes vor UV-Strahlung.

Die erfindungsgemäßen Zubereitungen können auch neben den Verbindungen der Formel I zusätzlich mindestens einen UV-Filter enthalten.

Organische UV-Filter, sogenannte hydrophile oder lipophile Sonnenschutzfilter, sind im UVA-Bereich und/oder UVB-Bereich und/oder IR und/oder VIS-Bereich (Absorber) wirksam. Diese Substanzen können insbesondere unter Zimtsäurederivaten, Salicylsäurederivaten, Campherderivaten, Triazinderivaten, β,β-Diphenylacrylatderivaten, p-Aminobenzoesäurederivaten sowie polymeren Filtern und Siliconfiltern, die in der Anmeldung WO-93/04665 beschrieben sind, ausgewählt sein. Weitere Beispiele für organische Filter sind in der Patentanmeldung EP-A 0 487 404 angegeben. Im Folgenden werden die genannten UV-Filter meist nach der INCI-Nomenklatur benannt.

Insbesondere für eine Kombination geeignet sind:
para-Aminobenzoesäure und deren Derivate: PABA, Ethyl PABA, Ethyl dihydroxypropyl PABA, Ethylhexyl dimethyl PABA, z. B. vertrieben unter dem Namen "Escalol 507" von der Fa. ISP, Glyceryl PABA, PEG-25 PABA, z. B. vertrieben unter dem Namen "Uvinul P25" von der Fa. BASF.

Salicylate: Homosalate vetrieben unter dem Namen "Eusolex HMS" von der Fa. Merck; Ethylhexyl salicylate, z. B. vertrieben unter dem Namen "Neo Heliopan OS" von der Fa. Symrise, Dipropylene glycol salicylate, z. B. vertrieben unter dem Namen "Dipsal" von der Fa. Scher, TEA salicylate, z. B. vertrieben unter dem Namen "Neo Heliopan TS" von der Fa. Symrise.

β,β-Diphenylacrylate Derivate: Octocrylene, z. B. vertrieben unter dem Namen "Eusolex® OCR" von der Firma Merck", "Uvinul N539" von der Fa. BASF, Etocrylene, z. B. vertrieben unter dem Namen "Uvinul N35" von der BASF.

Benzophenone Derivate: Benzophenone-1, z. B. vertrieben unter dem Namen "Uvinul 400"; Benzophenone-2, z. B. vertrieben unter dem Namen "Uvinul D50" ; Benzophenone-3 oder Oxybenzone, z. B. vertrieben unter dem Namen "Uvinul M40";Benzophenone-4, z. B. vertrieben unter dem Namen "Uvinul MS40" ; Benzophenone-9, z. B. vertrieben unter dem Namen "Uvinul DS-49" von der Fa. BASF, Benzophenone-5, Benzophenone-6, z. B. vertrieben unter dem Namen "Helisorb 11" von der Fa. Norquay, Benzophenone-8, z. B. vertrieben unter dem Namen "Spectra-Sorb UV-24" von der Fa. American Cyanamid, Benzophenone-12 n-hexyl 2-(4-diethylamino-2-hydroxybenzoyl) benzoate oder 2-Hydroxy-4-methoxybenzophenon, vertrieben von der Fa. Merck, Darmstadt unter dem Namen Eusolex® 4360.

Benzylidencampher Derivate: 3-Benzylidenecamphor, z. B. vertrieben unter dem Namen "Mexoryl SD" von der Fa. Chimex, 4-Methylbenzylidenecamphor, z. B. vertrieben unter dem Namen "Eusolex 6300" von der Fa. Merck, Benzylidenecamphorsulfonsäure, z. B. vertrieben unter dem Namen "Mexoryl SL" von der Fa. Chimex, Camphor benzalkonium methosulfate, z. B. vertrieben unter dem Namen "Mexoryl SO" von der Fa. Chimex, Terephthalylidenedicamphorsulfonsäure, z. B. vertrieben unter dem Namen "Mexoryl SX" von der Fa Chimex, Polyacrylamidomethylbenzylidenecamphor vertrieben unter dem Namen "Mexoryl SW" von der Fa. Chimex.

Phenylbenzimidazol Derivate: Phenylbenzimidazolesulfonsäure, z. B. vertrieben unter dem Namen "Eusolex 232" von der Fa. Merck, Dinatrium phenyl dibenzimidazole tetrasulfonat, z. B. vertrieben unter dem Namen "Neo Heliopan AP" von der Fa. Symrise.

Phenylbenzotriazol Derivate: Drometrizole trisiloxane, z. B. vertrieben unter dem Namen "Silatrizole" von der Fa. Rhodia Chimie, Methylenebis(benzotriazolyl)tetramethylbutylphenol in fester Form, z. B. vertrieben unter dem Namen "MIXXIM BB/100" von der Fa. Fairmount Chemical, oder in mikronisierter Form als wässrige Dispersion, z. B. vertrieben unter dem Namen "Tinosorb M" von der Fa. BASF.

Triazin Derivate: Ethylhexyltriazone, z. B. vertrieben unter dem Namen "Uvinul T150" von der Fa. BASF, Diethylhexylbutamidotriazone, z. B. vertrieben unter dem Namen "Uvasorb HEB" von der Fa. Sigma 3V, 2,4,6-tris(diisobutyl 4'-aminobenzalmalonate)-s-triazine oder 2,4,6-Tris-(biphenyl)-1,3,5-triazine vertrieben als Tinosorb A2B von BASF, 2,2'-[6-(4-methoxyphenyl)-1,3,5-triazine-2,4-diyl]bis[5-(2-ethylhexyl)oxy]-phenol, vertrieben als Tinosorb S von BASF, N2,N4-bis[4-[5-(1,1-dimethylpropyl)-2-benzoxazolyl]phenyl]-N6-(2-ethylhexyl)-1,3,5-triazin-2,4,6-triamin vertrieben als Uvasorb K 2A von der Firma Sigma 3V.

Anthranilin Derivate: Menthyl anthranilate, z. B. vertrieben unter dem Namen "Neo Heliopan MA" von der Fa. Symrise.

Imidazol Derivate: Ethylhexyldimethoxybenzylidenedioxoimidazoline propionat.

Benzalmalonat Derivate: Polyorganosiloxane enthaltend funktionelle benzalmalonate Gruppen, wie z.B. Polysilicone-15, z. B. vertrieben unter dem Namen "Parsol SLX" von der Hoffmann LaRoche.

4,4-Diarylbutadien Derivate: 1,1-Dicarboxy(2,2'-dimethylpropyl)-4,4-diphenylbutadiene.

Benzoxazole Derivate: 2,4-bis[5-(1-dimethylpropyl)benzoxazol-2-yl(4-phenyl) imino]-6-(2-ethylhexyl)imino-1,3,5-triazine, z. B. vertrieben unter dem Namen Uvasorb K2A von der Fa. Sigma 3V und Mischungen dieses enthaltend.

Piperazinderivate wie beispielsweise die Verbindung 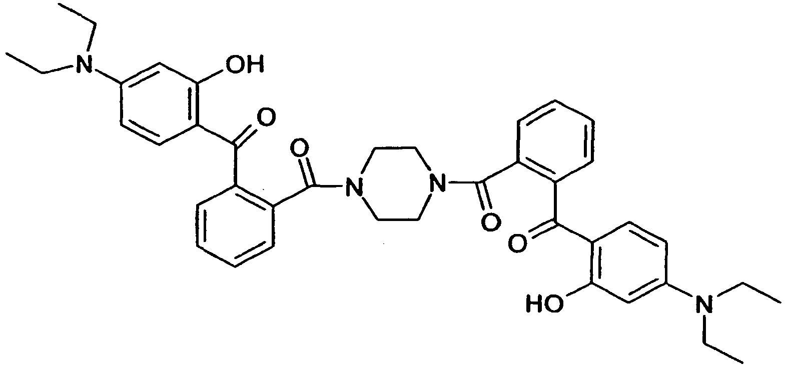 oder die UV-Filter der folgenden Strukturen 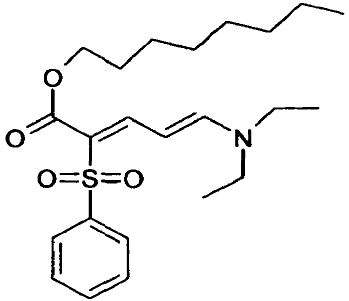 oder

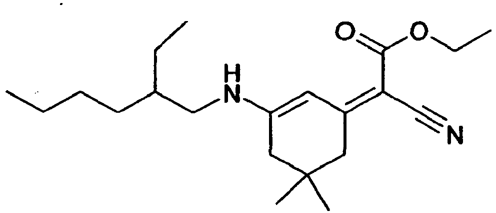

Es können auch UV-Filter auf Basis von Polysiloxancopolymeren mit einer statistischen Verteilung gemäß nachfolgender Formel verwendet werden, wobei z.B. a = 1,2; b= 58 und c=2,8 sind:

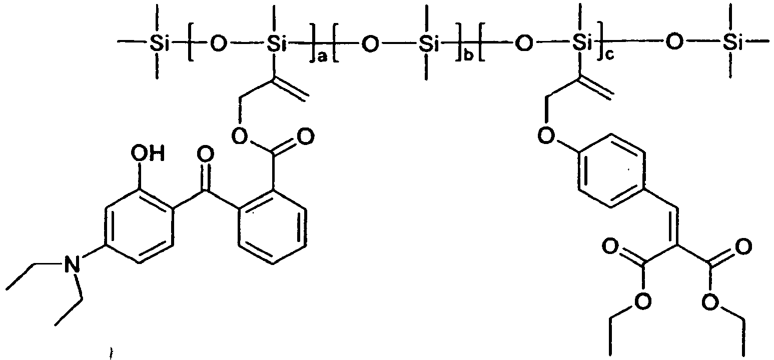

Die in der Liste aufgeführten Verbindungen sind nur als Beispiele aufzufassen. Selbstverständlich können auch andere UV-Filter verwendet werden.

Geeignete organischen UV-schützende Substanzen sind bevorzugt aus der folgenden Liste auszuwählen: Ethylhexyl salicylate, Phenylbenzimidazolesulfonic acid, Benzophenone-3, Benzophenone-4, Benzophenone-5, n-Hexyl 2-(4-diethylamino-2-hydroxybenzoyl)benzoate, 4-Methylbenzylidenecamphor, Terephthalylidenedicamphorsulfonic acid, Disodium phenyldibenzimidazoletetrasulfonate, Methylenebis(benzotriazolyl)tetramethylbutylphenol, Ethylhexyl Triazone, Diethylhexyl Butamido Triazone, Drometrizole trisiloxane, Polysilicone-15, 1,1-Dicarboxy(2,2'-dimethylpropyl)-4,4-diphenylbutadiene, 2,4-Bis[5-1 (dimethylpropyl)benzoxazol-2-yl(4-phenyl) imino]-6-(2-ethylhexyl)imino-1,3,5-triazine und Mischungen davon.

Diese organischen UV-Filter werden in der Regel in einer Menge von 0,01 Gewichtsprozent bis 20 Gewichtsprozent, vorzugsweise 1 Gew.-% - 10 Gew.-%, in Formulierungen eingearbeitet.

Die Zubereitungen können neben den Verbindungen der Formel I sowie den gegebenenfalls organischen UV-Filtern, wie zuvor beschrieben, weitere anorganische UV-Filter, sogenannte partikuläre UV-Filter, enthalten.

Diese Kombinationen mit partikulären UV-Filtern sind sowohl als Pulver als auch als Dispersion oder Paste der folgenden Typen möglich.

Hierbei sind sowohl solche aus der Gruppe der Titandioxide wie z.B. gecoatetes Titandioxid (z.B. Eusotex® T-2000, Eusolex®T-AQUA, Eusolex®T-AVO, Eusolex®T-OLEO), Zinkoxide (z.B. Sachtotec®), Eisenoxide oder auch Ceroxide und/oder Zirkonoxide bevorzugt.

Ferner sind auch Kombinationen mit pigmentärem Titandioxid oder Zinkoxid möglich, wobei die Partikelgröße dieser Pigmente größer oder gleich 200 nm sind, beispielsweise Hombitan® FG oder Hombitan® FF-Pharma.

Weiter kann es bevorzugt sein, wenn die Zubereitungen anorganische UV-Filter enthalten, die mit üblichen Methoden, wie beispielsweise in Cosmetics & Toiletries, February 1990, Vol. 105, pp. 53 64 beschrieben, nachbehandelt wurden. Hierbei können eine oder mehrere der folgenden Nachbehandlungskomponenten gewählt sein: Amino Säuren, Bienenwachs, Fettsäuren, Fettsäurealkohole, anionische Tenside, Lecithin, Phospholipide, Natrium-, Kalium-, Zink-, Eisen- oder Aluminiumsalze von Fettsäuren, Polyethylene, Silikone, Proteine (besonders Collagen oder Elastin), Alkanolamine, Siliciumdioxid, Aluminiumoxid, weitere Metalloxide, Phosphate, wie Natriumhexametaphosphat oder Glycerin.

Bevorzugt eingesetzte partikuläre UV-Filter sind dabei:
- unbehandelte Titandioxide wie z.B. die Produkte Microtitanium Dioxide MT 500 B der Fa. Tayca; Titandioxd P25 der Fa. Degussa,
- Nachbehandelte mikronisierte Titandioxide mit Aluminiumoxid und Siliciumdioxid Nachbahandlung wie z.B. das Produkt "Microtitanium Dioxide MT 100 SA der Tayca; oder das Produkt "Tioveil Fin" der Fa. Uniqema,
- Nachbehandelte mikronisierte Titandioxide mit Aluminiumoxid und/oder Aluminiumstearate/laurate Nachbehandlung wie z.B. Microtitanium Dioxide MT 100 T der Fa. Tayca, Eusolex T-2000 der Firma Merck,
- Nachbehandelte mikronisierte Titandioxide mit Eisenoxid und/oder Eisenstearate Nachbehandlung wie z.B. das Produkt "Microtitanium Dioxide MT 100 F" der Fa. Tayca,
- Nachbehandelte mikronisierte Titandioxide mit Siliciumdioxide, Aluminiumoxid und Silicon Nachbehandlung wie z.B. das Produkt "Microtitanium Dioxide MT 100 SAS",der Fa. Tayca,
- Nachbehandelte mikronisierte Titandioxide mit Natrumhexameta¬phosphate, wie z.B. das Produkt "Microtitanium Dioxide MT 150 W" der Fa. Tayca.

Die zur Kombination eingesetzten behandelten mikronisierten Titandioxide können auch nachbehandelt sein mit:
- Octyltrimethoxysilane; wie z.B. das Produkt Tego Sun T 805 der Fa. Degussa,
- Siliciumdioxid; wie z.B. das Produkt Parsol T-X der Fa. DSM,
- Aluminiumoxid und Stearinsäure; wie z.B. das Produkt UV-Titan M160 der Fa. Sachtleben,
- Aluminium und Glycerin; wie z.B. das Produkt UV-Titan der Fa. Sachtleben
- Aluminium und Silikonölen, wie z.B. das Produkt UV-Titan M262 der Fa. Sachtleben,
- Natriumhexamethaphosphat und Polyvinylpyrrolidon,
- Polydimethylsiloxane, wie z.B. das Produkt 70250 Cardre UF TiO2SI3" der Fa. Cardre,
- Polydimethylhydrogensiloxane, wie z.B. das Produkt Microtitanium Dioxide USP Grade Hydrophobic" der Fa. Color Techniques.

Ferner kann auch die Kombination mit folgenden Produkten vorteilhaft sein:
- Unbehandelte Zinkoxide wie z. B. das Produkt Z-Cote der Fa. BASF (Sunsmart), Nanox der Fa. Elementis
- Nachbehandelte Zinkoxide wie z.B die folgenden Produkte:
   - "Zinc Oxide CS-5" der Fa. Toshibi (ZnO nachbehandelt mit polymethylhydrogenosiloxane)
   - Nanogard Zinc Oxide FN der Fa. Nanophase Technologies
   - "SPD-Z1" der Fa Shin-Etsu (ZnO nachbehandelt mit einem Silikongepfropften Acrylpolymer, dispergiert in Cyclodimethyl-siloxane
   - "Escalol Z100" der Fa ISP (Aluminiumoxid nachbehandeltes ZnO dispergiert in einer ethylhexyl methoxycinnamate/PVP-hexadecene/ methicone copolymer Mischung)
   - "Fuji ZNO-SMS-10" der Fa. Fuji Pigment (ZnO nachbehandelt mit Siliciumdioxid und Polymethylsilesquioxan);
   - Unbehandeltes Ceroxide Mikropigment z.B. mit der Bezeichnung "Colloidal Cerium Oxide" der Fa Rhone Poulenc
   - Unbehandelte und/oder nachbehandelte Eisenoxide mit der Bezeichnung Nanogar der Fa. Arnaud.

Beispielhaft können auch Mischungen verschiedener Metalloxide , wie z.B. Titandioxid und Ceroxid mit und ohne Nachbenhandlung eingesetzt werden, wie z.B. das Produkt Sunveil A der Fa. Ikeda. Außerdem können auch Mischungen von Aluminiumoxid, Siliciumdioxid und Silikonnachbehandeltem Titandioxid, Zinkoxid-Mischungen wie z.B. das Produkt UV-Titan M261 der Fa. Sachtleben verwendet werden.

Diese anorganischen UV-Filter werden in der Regel in einer Menge von 0,1 Gewichtsprozent bis 25 Gewichtsprozent, vorzugsweise 2 Gew.-% - 10 Gew.-%, in die Zubereitungen eingearbeitet.

Durch Kombination von einer oder mehrerer der genannten Verbindungen mit UV-Filterwirkung kann die Schutzwirkung gegen schädliche Einwirkungen der UV-Strahlung optimiert werden.

Alle genannten UV-Filter können auch in verkapselter Form eingesetzt werden. Insbesondere ist es von Vorteil organische UV-Filter in verkapselter Form einzusetzen.

Dabei sind die Kapseln in erfindungsgemäß einzusetzenden Zubereitungen vorzugsweise in solchen Mengen enthalten, die gewährleisten, dass die verkapselten UV-Filter in den oben angegebenen Gewichtsprozentverhältnissen in der Zubereitung vorliegen.

In den beschriebenen Zubereitungen, die erfindungsgemäß mindestens eine Verbindung der Formel I enthalten, können weiterhin auch Farbpigmente enthalten sein, wobei der Schichtaufbau der Pigmente nicht limitiert ist.

Vorzugsweise sollte das Farbpigment bei Einsatz von 0,5 bis 5 Gew.-% hautfarben oder bräunlich sein. Die Auswahl eines entsprechenden Pigments ist für den Fachmann geläufig.

Bevorzugte Zubereitungen können ebenfalls mindestens einen weiteren kosmetischen Wirkstoff enthalten, beispielsweise ausgewählt aus Antioxidantien, Anti-ageing-, Anti-Falten-, Anti-Schuppen-, Anti-Akne-, Anti-Cellulite-Wirkstoffen, Deodorants oder Vitaminen.

Die schützende Wirkung von Zubereitungen gegen oxidativen Stress bzw. gegen die Einwirkung von Radikalen kann verbessert werden, wenn die Zubereitungen ein oder mehrere Antioxidantien enthalten, wobei es dem Fachmann keinerlei Schwierigkeiten bereitet geeignet schnell oder zeitverzögert wirkende Antioxidantien auszuwählen.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA, Pentasodium ethylenediamin tetramethylen phosphonat und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, ZnSO₄), Selen und dessen Derivate (z.B. Selen¬methionin), Stilbene und deren Derivate (z.B. Stilbenoxid, trans-Stilbenoxid).

Geeignete Antioxidantien sind auch Verbindungen der Formeln A oder B 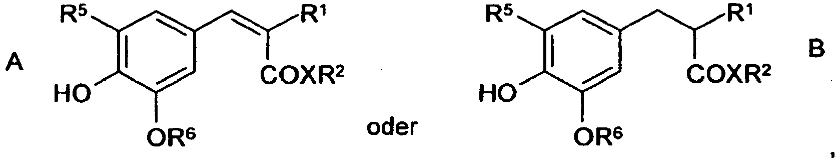 worin
- R¹: aus der Gruppe -C(O)CH₃, -CO₂R³, -C(O)NH₂ und -C(O)N(R⁴)₂ ausgewählt werden kann,
- X: O oder NH,
- R²: lineares oder verzweigtes Alkyl mit 1 bis 30 C-Atomen,
- R³: lineares oder verzweigtes Alkyl mit 1 bis 20 C-Atomen,
- R⁴: jeweils unabhängig voneinander H oder lineares oder verzweigtes Alkyl mit 1 bis 8 C-Atomen,
- R⁵: H, lineares oder verzweigtes Alkyl mit 1 bis 8 C-Atomen oder lineares oder verzweigtes Alkoxy mit 1 bis 8 C-Atomen und
- R⁶: lineares oder verzweigtes Alkyl mit 1 bis 8 C-Atomen bedeutet,
vorzugsweise Derivate der 2-(4-Hydroxy-3,5-dimethoxybenzyliden)-malonsäure und/oder 2-(4-Hydroxy-3,5-dimethoxybenzyl)-malonsäure, besonders bevorzugt 2-(4-Hydroxy-3,5-dimethoxybenzyliden)-malonsäure-bis-(2-ethylhexyl)ester (z.B. Oxynex® ST Liquid) und/oder 2-(4-Hydroxy-3,5-dimethoxybenzyl)-malonsäure-bis-(2-ethylhexyl)ester (z.B. RonaCare® AP).

Mischungen von Antioxidantien sind ebenfalls zur Verwendung in den erfindungsgemäßen kosmetischen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure, natürliche Tocopherole, L-(+)-Ascorbylpalmitat, L-(+)-Ascorbinsäure und Zitronensäure (z.B. Oxynex® K LIQUID), Tocopherolextrakte aus natürlichen Quellen, L-(+)-Ascorbylpalmitat, L-(+)-Ascorbinsäure und Zitronensäure (z.B. Oxynex® L LIQUID), DL-α-Tocopherol, L (+)-Ascorbylpalmitat, Zitronensäure und Lecithin (z.B. Oxynex® LM) oder Butylhydroxytoluol (BHT), L-(+)-Ascorbylpalmitat und Zitronensäure (z.B. Oxynex® 2004). Derartige Antioxidantien werden mit den erfindungsgemäßen Verbindungen in solchen Zusammensetzungen üblicherweise in Gewichtsprozentverhältnissen im Bereich von 1000:1 bis 1:1000, bevorzugt in Gewichtsprozentverhältnissen von 100:1 bis 1:100 eingesetzt.

Unter den Phenolen, die erfindungsgemäß verwendet werden können, sind die teilweise als Naturstoffe vorkommenden Polyphenole für Anwendungen im pharmazeutischen, kosmetischen oder Ernährungsbereich besonders interessant. Beispielsweise weisen die hauptsächlich als-Pflanzenfarbstoffe bekannten Flavonoide oder Bioflavonoide häufig ein antioxidantes Potential auf. Mit Effekten des Substitutionsmusters von Mono- und Dihydroxyflavonen beschäftigen sich K. Lemanska, H. Szymusiak, B. Tyrakowska, R. Zielinski, I.M.C.M. Rietjens; Current Topics in Biophysics 2000, 24(2), 101-108. Es wird dort beobachtet, dass Dihydroxyflavone mit einer OH-Gruppe benachbart zur Ketofunktion oder OH Gruppen in 3'4'-oder 6,7- oder 7,8-Position antioxidative Eigenschaften aufweisen, während andere Mono- und Dihydroxyflavone teilsweise keine antioxidativen Eigenschaften aufweisen.

Häufig wird Quercetin (Cyanidanol, Cyanidenolon 1522, Meletin, Sophoretin, Ericin, 3,3',4',5,7-Pentahydroxyflavon) als besonders wirksames Antioxidans genannt (z.B. C.A. Rice-Evans, N.J. Miller, G. Paganga, Trends in Plant Science 1997, 2(4), 152-159). K. Lemanska, H. Szymusiak, B. Tyrakowska, R. Zielinski, A.E.M.F. Soffers und I.M.C.M. Rietjens (Free Radical Biology&Medicine 2001, 31(7), 869-881 untersuchen die pH-Abhängigkeit der antioxidanten Wirkung von Hydoxyflavonen. Über den gesamten pH-Bereich zeigt Quercetin die höchste Aktivität der untersuchten Strukturen.

Geeignete anti-ageing Wirkstoffe, insbesondere für hautpflegende Zubereitungen, sind vorzugsweise sogenannte kompatible Solute. Es handelt sich dabei um Substanzen, die an der Osmoregulation von Pflanzen oder Mikroorganismen beteiligt sind und aus diesen Organismen isoliert werden können. Unter den Oberbegriff kompatible Solute werden dabei auch die in der Deutschen Patentanmeldung DE-A-10133202 beschriebenen Osmolyte gefasst. Geeignete Osmolyte sind beispielsweise die Polyole, Methylamin- Verbindungen und Aminosäuren sowie jeweils deren Vorstufen. Als Osmolyte werden im Sinne der Deutschen Patentanmeldung DE-A-10133202 insbesondere Substanzen aus der Gruppe der Polyole, wie beispielsweise myo-Inositol, Mannitol oder Sorbitol und/oder einer oder mehrere der nachfolgend genannten osmolytisch wirksamen Stoffe verstanden: Taurin, Cholin, Betain, Phosphorylcholin, Glycerophosphorylcholine, Glutamin, Glycin, α-Alanin, Glutamat, Aspartat, Prolin, und Taurin. Vorstufen dieser Stoffe sind beispielsweise Glucose, Glucose-Polymere, Phosphatidylcholin, Phosphatidylinositol, anorganische Phosphate, Proteine, Peptide und Polyaminsäuren. Vorstufen sind z. B. Verbindungen, die durch metabolische Schritte in Osmolyte umgewandelt werden.

Vorzugsweise werden erfindungsgemäß als kompatible Solute Substanzen gewählt aus der Gruppe bestehend aus Pyrimidincarbonsäuren (wie Ectoin und Hydroxyectoin), Prolin, Betain, Glutamin, cyclisches Diphosphoglycerat, N.-Acetylornithin, Trimethylamine-N-oxid Di-myoinositol-phosphat (DIP), cyclisches 2,3-diphosphoglycerat (cDPG), 1,1-Diglycerin-Phosphat (DGP), ß-Mannosylglycerat (Firoin), β-Mannosylglyceramid (Firoin-A) oder/und Di-mannosyl-di-inositolphosphat (DMIP) oder ein optisches Isomer, Derivat, z.B. eine Säure, ein Salz oder Ester dieser Verbindungen oder Kombinationen davon eingesetzt.

Dabei sind unter den Pyrimidincarbonsäuren insbesondere Ectoin ((S)-1,4,5,6-Tetrahydro-2-methyl-4-pyrimidincarbonsäure) und Hydroxyectoin ((S,S)-1,4,5,6-Tetrahydro-5-hydroxy-2-methyl-4-pyrimidincarbonsäure) und deren Derivate zu nennen.

Additional können als anti-ageing Wirkstoffe Produkte der Firma Merck wie z.B. 5,7-Dihydroxy-2-methyl-chromon, vermarktet unter dem Handelsnamen RonaCare®Luremine, Ronacare®Isoquercetin, Ronacare®Tilirosid oderRonacare®Cyclopeptide 5 verwendet werden.

Die einzusetzenden Zubereitungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin B₁), Riboflavin (Vitamin B₂), Nicotinsäureamid, Vitamin C (Ascorbinsäure), Vitamin D, Ergocalciferol (Vitamin D₂), Vitamin E, DL-α-Tocopherol, Tocopherol-E-Acetat, Tocopherolhydrogensuccinat, Vitamin K₁, Esculin (Vitamin P-Wirkstoff), Thiamin (Vitamin B₁). Nicotinsäure (Niacin), Pyridoxin, Pyridoxal, Pyridoxamin, (Vitamin B₆), Panthothensäure, Biotin, Folsäure und Cobalamin (Vitamin B₁₂), insbesondere bevorzugt Vitamin-A-Palmitat, Vitamin C und dessen Derivate, DL-α-Tocopherol, Tocopherol-E-Acetat, Nicotinsäure, Pantothensäure und Biotin. Vitamine werden mit den flavonoidhaltigen Vormischungen oder Zubereitungen üblicherweise bei kosmetischer Anwendung in Bereichen von 0,01 bis 5,0 Gew.-%, bezogen auf das Gesamtgewicht, zugesetzt. Ernährungsphysiologische Anwendungen orientieren sich am jeweiligen empfohlenen Vitaminbedarf.

Die beschriebenen Retinoide sind gleichzeitig auch wirksame Anti-Cellulite-Wirkstoffe. Ein ebenfalls bekannter Anti-Cellulite-Wirkstoff ist Koffein.

Gegenstand der vorliegenden Erfindung ist auch ein Verfahren zur Herstellung einer Zubereitung, wie zuvor beschrieben, dadurch gekennzeichnet, dass mindestens eine Verbindung der Formel I mit einem für topische Zubereitungen geeigneten Träger und optional mit Hilfs- und oder Füllstoffen vermischt wird. Geeignete Trägerstoffe sowie Hilfs- oder Füllstoffe sind im nachfolgenden Teil ausführlich beschrieben.

Die genannten Bestandteile der Zubereitung können in der üblichen Weise eingearbeitet werden, mit Hilfe von Techniken, die dem Fachmann wohl bekannt sind.

Die kosmetischen und dermatologischen Zubereitungen können in verschiedenen Formen vorliegen. So können sie z. B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsion, beispielsweise vom Typ Waser-in-Öl-in-Wasser (W/O/W) oder O/W/O, ein Gel, einen festen Stift, eine Salbe oder auch ein Aerosol darstellen. Bevorzugt werden Emulsionen. O/W-Emulsinen werden besonders bevorzugt. Emulsionen, W/O-Emulsionen und O/W-Emulsionen sind in üblicher Weise erhältlich.

Als Anwendungsform der einzusetzenden Zubereitungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole, Pflaster, Umschläge, Verbände und Sprays.

Bevorzugte Hilfsstoffe stammen aus der Gruppe der Konservierungsstoffe, Stabilisatoren, Lösungsvermittler, Färbemittel, Geruchsverbesserer.

Salben, Pasten, Cremes und Gele können die üblichen Trägerstoffe enthalten, die für die topische Verabreichung geeignet sind, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen leichtflüchtigen, verflüssigten Treibmittel, z.B. Chlorfluorkohlenwasserstoffe, Propan/Butan oder Dimethylether, enthalten. Auch Druckluft ist vorteilhaft zu verwenden.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethylacetat, Benzylalkohol, Benzylbenzoat, Propylenglykol, 1,3 Butylglykol, Öle, insbesondere Baumwollsaatöl, Erdnussöl, Maiskeimöl, Olivenöl, Rizinusöl und Sesamöl, XTend 226 (L'Oréal), Glycerinfettsäureester, Polyethylenglykole und Fettsäureester des Sorbitans oder Gemische dieser Stoffe enthalten.

Ein bevorzugter Lösungsvermittler generell ist 2-Isopropyl-5-methyl-cyclohexancarbonyl-D-Alaninmethylester.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typische kosmetische Anwendungsformen sind auch Lippenstifte, Lippenpflegestifte, Puder-, Emulsions- und Wachs-Make up sowie Sonnenschutz-, Prä-Sun- und After-Sun-Präparate.

Zu den bevorzugten Zubereitungsformen gehören insbesondere auch Emulsionen.

Emulsionen sind vorteilhaft und enthalten z. B. die genannten Fette, Öle, Wachse und anderen Fettkörper, sowie Wasser und einen Emulgator, wie er üblicherweise für einen solchen Typ der Zubereitung verwendet wird.

Die Lipidphase kann vorteilhaft gewählt werden aus folgender Substanzgruppe:
- Mineralöle, Mineralwachse
- Öle, wie Triglyceride der Caprin oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C Zahl, z.B. mit Isopropanol, Propylenglykol oder Glycerin, oder Ester von Fettalkoholen mit Alkansäuren niedriger C Zahl oder mit Fettsäuren;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigtem und/oder ungesättigten, verzweigten und/oder unverzweigten Alkancarbonsäuren einer Kettenlänge von 3 bis 30 C Atomen und gesättigten und/oder ungesättigten, verzweigten und/oder unverzweigten Alkoholen einer Kettenlänge von 3 bis 30 C Atomen, aus der Gruppe der Ester aus aromatischen Carbonsäure und gesättigten und/oder ungesättigten, verzweigten und/oder unverzweigten Alkoholen einer Kettenlänge von 3 bis 30 C Atomen. Solche Esteröle können dann vorteilhaft gewählt werden aus der Gruppe Isopropylmyristat, Isopropylpalmitat, Isopropylstearat, Isopropyloleat, n Butylstearat, n-Hexyllaurat, n Decyloleat, Isooctylstearat, Isononylstearat, Isononylisononanoat, 2 Ethylhexylpalmitat, 2 Ethylhexyllaurat, 2 Hexaldecylstearat, 2 Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Die wässrige Phase der einzusetzenden Zubereitungen enthält gegebenenfalls vorteilhaft Alkohole, Diole oder Polyole niedriger C Zahl, sowie deren Ether, vorzugsweise Ethanol, Isopropanol, Propylenglykol, Glycerin, Ethylenglykol, Ethylenglykolmonoethyl oder -monobutylether, Propylenglykolmonomethyl, -monoethyl oder -monobutylether, Diethylenglykolmonomethyl oder -monoethylether und analoge Produkte, ferner Alkohole niedriger C Zahl, z. B. Ethanol, Isopropanol, 1,2 Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

In einer bevorzugten Ausführungsform enthalten die einzusetzenden Zubereitungen hydrophile Tenside. Die hydrophilen Tenside werden bevorzugt gewählt aus der Gruppe der Alkylglucoside, der Acyllactylate, der Betaine sowie der Cocoamphoacetate.

Als Emulgatoren können beispielsweise die bekannten W/O- und O/W-Emulgatoren verwendet werden. Es ist vorteilhaft, weitere übliche Co-Emulgatoren in den bevorzugten O/W-Emulsionen zu verwenden.

Vorteilhaft werden als Co-Emulgatoren beispielsweise O/W-Emulgatoren gewählt, vornehmlich aus der Gruppe der Substanzen mit HLB-Werten von 11-16, ganz besonders vorteilhaft mit HLB-Werten von 14,5-15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkhole, Cetylalkohole, Cetylstearylalkohole (Cetearylalkohole) zu wählen.

Es ist ferner von Vorteil, die Fettsäureethoxylate aus folgender Gruppe zu wählen:
Polyethylenglycol(20)stearat, Polyethylenglycol(21)stearat,
Polyethylenglycol(22)stearat, Polyethylenglycol(23)stearat,
Polyethylenglycol(24)stearat, Polyethylenglycol(25)stearat,
Polyethylenglycol(12)isostearat, Polyethylenglycol(13)isostearat,
Polyethylenglycol(14)isostearat, Polyethylenglycol(15)isostearat,
Polyethylenglycol(16)isostearat, Polyethylenglycol(17)isostearat,
Polyethylenglycol(18)isostearat, Polyethylenglycol(19)isostearat,
Polyethylenglycol(20)isostearat, Polyethylenglycol(21)isostearat,
Polyethylenglycol(22)isostearat, Polyethylenglycol(23)isostearat,
Polyethylenglycol(24)isostearat, Polyethylenglycol(25)isostearat,
Polyethylenglycol(12)oleat, Polyethylenglycol(13)oleat,
Polyethylenglycol(14)oleat, Polyethylenglycol(15)oleat,
Polyethylenglycol(16)oleat, Polyethylenglycol(17)oleat,
Polyethylenglycol(18)oleat, Polyethylenglycol(19)oleat,
Polyethylenglycol(20)oleat.

Als ethoxylierte Alkylethercarbonsäure bzw. deren Salz kann vorteilhaft das Natriumlaureth-11-carboxylat verwendet werden. Als Alkylethersulfat kann Natrium Laureth1-4sulfat vorteilhaft verwendet werden. Als ethoxyliertes Cholesterinderivat kann vorteilhaft Polyethylenglycol(30)Cholesterylether verwendet werden. Auch Polyethylenglycol(25)Sojasterol hat sich bewährt. Als ethoxylierte Triglyceride können vorteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/cprinat, Polyethylenglycol(20)glyceryloleat, Polyethylenglycol(20)glycerylisostearat, Polyethylenglycol(18)glyceryloleat(cocoat) zu wählen. Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als fakultative, dennoch erfindungsgemäß gegebenenfalls vorteilhafte W/O-Emulgatoren können eingesetzt werden:
Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12-18 C Atome, Diglycerinester gesättigter und/oder ungesättigter, verzweigter und/oder unverzweigter Alkancarbonsäuren einer Kettenlänge von 8 bis 24, insbesondere 12-18 C¬ Atomen, Monoglycerinether gesättigter und/oder ungesättigter, verzweigter und/oder unverzweigter Alkohole einer Kettenlänge von 8 bis 24, insbesondere 12-18 C-Atomen, Diglycerinether gesättigter und/oder ungesättigter, verzweigter und/oder unverzweigter Alkohole einer Kettenlänge von 8 bis 24, insbesondere 12-18 C Atomen, Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12-18 C-Atomen.

Insbesondere vorteilhafte W/O-Emulgatoren sind Glycerylmonostearat, Glycerylmonoisostearat, Glycerylmonomyristat, Glycerylmonooleat, Diglyceryimonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat oder PEG-30-dipolyhydroxystearat.

Die Zubereitung kann kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Eine bevorzugte Ausführungsform der Erfindung ist eine Emulsion, welche als Schutzcreme oder -milch vorliegt und beispielsweise Fettalkohole, Fettsäuren, Fettsäureester, insbesondere Triglyceride von Fettsäuren, Lanolin, natürliche und synthetische Öle oder Wachse und Emulgatoren in Anwesenheit von Wasser enthält.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder öligalkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Die Zubereitung kann auch als alkoholisches Gel vorliegen, welches einen oder mehrere Niedrigalkohole oder -polyole, wie Ethanol, Propylenglykol oder Glycerin, und ein Verdickungsmittel, wie Kieselerde umfasst. Die öligalkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane , bevorzugt Alkane.

Auch ohne weitere Ausführungen wird davon ausgegangen, dass ein Fachmann die obige Beschreibung in weitestem Umfang nutzen kann. Die bevorzugten Ausführungsformen und Beispiele sind deswegen lediglich als beschreibende, keinesfalls als in irgendeiner Weise limitierende Offenbarung aufzufassen. Die vollständige Offenbarung aller vor- und nachstehend aufgeführten Anmeldungen und Veröffentlichungen sind durch Bezugnahme in diese Anmeldung eingeführt.

Weitere wichtige Merkmale und Vorteile der Erfindung ergeben sich aus den Unteransprüchen und aus den Beispielen.

Die Beispiele sollen die vorliegende Erfindung näher erläutern, ohne deren Umfang zu beschränken.

### Beispiele:

### Beispiel 1:

### Synthese von Hexadecansäure 5-hydroxy-2-methyl-4-oxo-4H-chromen-7-yl ester (Ia)

5,7-Dihydroxymethylchromon (15 g, 78 mmol) wird in N,N-dimethylformamid (360 mL) gelöst und Kalium-tert-butylat (9 g, 80 mmol) unter Stickstoffstrom zu der braunen Lösung hinzugefügt. Anschließend wird für 2,5 h auf 30°C erhitzt. Dann wird Palmitinsäurechlorid (24 ml, 79,5mmol) in N,N-dimethylformamide (80 ml) langsam bei 30°C zu der Lösung zugetropft. Die entstehende Suspension wird noch 2 Stunden bei 30°C weitergerührt und dann 400 ml VE-Wasser zugetropft. Der Feststoff wird nach 1 Stunde über eine Nutsche abgesaugt, gut trocken gesaugt und im Vakuumtrockenschrank bei 40°C getrocknet.
Ausbeute (nach Umkristallisierung in Ethanol): 22g =66% der Theorie Farbe: beige
Summenformel: C₂₆H₃₈O₅
Molmasse: 430,6 g/mol

### Analytik:

MS (EI): m/z (Relative Intensität, %) = 430,2 ([M+] 25), 192,1 ([M+] 100). 1H-NMR (CDCl₃, 500MHz): δ = 12.28 (s, 1H, OH), 6.68 (d, 1H, J=2Hz), 6.51 (d, 1H, *J*=2Hz), 6.1 (s, 1H), 2.56 (t, 2H, *J*=7.5), 1.75 (m, 2H), 1.26 (m, 24H), 0.88 (t, 3H, *J*=6.7Hz).

### Beispiel 2:

### Synthese von (E)-3,7-Dimethyl-octa-2,6-diensäure 5-hydroxy-2-methyl-4-oxo-4H-chromen-7-yl ester (Ib)

Analog zu Beispiel 1 wird die Verbindung Ib hergestellt. Anstelle von Palmitinsäurechlorid wird 3,7-Dimethyl-octa-2,6-diensäurechlorid eingesetzt.

### Analytik:

Summenformel: C₂₀H₂₂O₅
Molmasse: 342,4 g/mol
MS (EI): m/z (Relative Intensität, %) = 342 ([M+] 15), 69 ([M+] 100). 1H-NMR (DMSO-δ₆, 300MHz): δ = 12.86 (s, 1H, OH), 6.9 (d, 1 H), 6.62 (d, 1 H), 6.35 (s, 1 H), 5.93 (s, 1 H), 5.11 (t, 1 H), 2.40 (s, 3H), 2.26-2.17 (m, 7H), 1.67 (s, 3H), 1.60 (s, 3H).

### Beispiel 3:

### Synthese von (E)-3-(4-Hydroxy-3-methoxyphenyl)-acrylsäure 5-hydroxy-2-methyl-4-oxo-4H-chromen-7-yl ester (Ic)

Analog zu Beispiel 1 wird die Verbindung Ic hergestellt. Anstelle von Palmitinsäurechlorid wird 4-Hydroxy-3-methoxyzimtsäurechlorid eingesetzt.

### Beispiel 4:

### Synthese von (E)-Octadec-9-ensäure 5-hydroxy-2-methyl-4-oxo-4H-chromen-7-yl ester (Id)

Analog zu Beispiel 1 wird die Verbindung Id hergestellt. Anstelle von Palmitinsäurechlorid wird (E)-Octadec-9-ensäurechlorid eingesetzt.

### Analytik:

Summenformel: C₂₈H₄₀O₅
Molmasse: 456,63 g/mol
MS (EI): m/z (Relative Intensität, %) = 456 ([M+] 25), 192 ([M+] 100). 1H-NMR (DMSO-δ₆, 500MHz): δ = 15.49 (s, 1H, OH), 6.78 (d, 1 H), 6.57 (d, 1 H), 6.23 (s, 1 H), 5.38 (t, 2H), 2.59 (t, 2H), 2.38 (s, 3H), 2.04 (t, 4H), 1.71 (q, 2H), 1.45-1.22 (m, 20H), 0.86 (t, 3H)

### Beispiel 5:

### Ex vivo Studie zur Promotion der Melanogenese

### Präparation der Gewebeproben

Die Gewebeproben mit 10mm Durchmesser werden aus dem Bauchgewebe einer 32 jährigen Frau vom kaukasischen Typus entnommen und bei 37°C in einer feuchten Atmosphäre mit einem Gehalt von 5% CO₂ in BEM-Medium gelagert. Die Gewebeproben werden gemäß folgendem Testplan eingesetzt:

| Probe | Topische Anwendung | UV | Zahl der Gewebeproben |
|---|---|---|---|
| Unbehandelt Haut (T0) | - | ohne | 3 |
| Unbehandelte Haut | - | ohne | 6 |
| Placebo | ja | ohne | 6 |
| la | ja | ohne | 6 |
| 5-Hydroxy-7-acetyl-2-methyl-chromen-4-on (Vergleichssubstanz) | ja | ohne | 6 |
| 5-Hydroxy-7-methoxy-2-methyl-chromen-4-on (Vergleichssubstanz) | ja | ohne | 6 |
| Unbehandelte Haut | - | mit | 6 |
| Placebo | ja | mit | 6 |
| la | ja | mit | 6 |
| 5-Hydroxy-7-acetyl-2-methyl-chromen-4-on (Vergleichssubstanz) | ja | mit | 6 |
| 5-Hydroxy-7-methoxy-2-methyl-chromen-4-on (Vergleichssubstanz) | ja | mit | 6 |

### Produktapplikation

30µL Testlösung werden auf ein Filterpapier gegeben und sofort auf die Gewebeprobe gelegt.

### Bestrahlungen

Die Bestrahlungen werden täglich vor Behandlung der Gewebeproben mit Testlösungen durchgeführt. Es wird ein RMX 3W Vilbert Lourmat Gerät verwendet. Die UV-Dosis beträgt 1.04 J/cm2 mit 6/8% UVB.

### Probennahme

Am ersten Tag (D0) werden 3 unbehandelte Gewebeproben halbiert und jeweils eine Hälfte in gepufferter Formalinlösung fixiert. Die jeweils anderen Hälften werden bei -80°C eingefroren.

### Histologische Analyse

Nach einer 24 stündigen Fixierung in gepufferter Formalinlösung werden die Proben in einem Leica TP1010 Trocknungsautomaten dehydratisert und mit Paraffin imprägniert. Nach Einbettung mit Hilfe einer Leica EG1160 Station werden aus den Proben mit einem Leica RM 2125 Minot-Typ Mikrotom 5µm dünne Querschnitte hergestellt. Die Querschnitte werden auf Superfrost Plus Objektträger aus silanisiertem Glas befestigt. Die lichtmikroskopische Analyse erfolgt mittels eines Leica Orthoplan Mikroskops mit 25-facher Vergrößerung zur Begutachtung der generellen Morphologie bzw. einer 40-fachen Vergrößerung zur Visualisierung des Melanins.

### Generelle Morphologie

Die Begutachtung der generellen Morphologie erfolgt nach Einfärbung der paraffinierten Querschnitte nach Masson's trichrome in der Goldner Variante.

### Melaninvisualisierung

Die Melaninvisualisierung erfolgt nach Imprägnierung der paraffinierten Querschnitte mit Silber nach Masson in der Fontana Variante.

### Chromatometrie

Die Bestimmung der Farbkoordinaten der Gewebeproben erfolgt mittels eines Minolta CM 2600d Chromatometers.

### Ergebnisse

### 1) Testserie ohne UV-Bestrahlung:

| | | L | %L |
|---|---|---|---|
| Unbehandelte Probe | D0 | 61.41 | |
| | D10 | 62.22 | 1.3 |
| Mit Placebo behandelte Probe | D0 | 61.68 | |
| | D10 | 63.05 | 2.2 |
| Mit Verbindung la behandelte Probe | D0 | 64.53 | |
| | D10 | 61.24 | -5.1 |
| Mit 5-Hydroxy-7-acetyl-2-methyl-chromen-4-on behandelte Probe | D0 | 62.19 | |
| | D10 | 63.98 | 2.9 |
| Mit 5-Hydroxy-7-methoxy-2-methyl-chromen-4-on behandelte Probe | D0 | 61.50 | |
| | D10 | 64.64 | 5.1 |

### Unbehandelte Proben:

Nach 10 tägiger Behandlung zeigen die Melanocyten einen moderaten Grad an dendritischer Verzweigung und Beladung mit Melanin. Die Zahl an Kontakten mit benachbarten Keratinocyten ist gering, ebenso die Melanintransferrate. Der Gehalt an Melanin in basalen Keratinocyten ist moderat und niedrig in suprabasalen Keratinozyten.

### Mit Placebo behandelte Proben:

Die Melanocyten zeigen nach 10 tägiger Behandlung eine geringe Aktivität. Melanin ist in basalen Keratinocyten klar nachweisbar, der Melaningehalt in suprabasalen Keratinocyten ist gering.

### Mit Verbindung la behandelte Proben:

Die Melanocyten zeigen nach 10 tägiger Behandlung eine sehr hohe Aktivität. Melamin ist gut nachweisbar in basalen Keratinocyten, der Melaningehalt in suprabasalen Keratinocyten ist moderat.

### Mit 5-Hydroxy-7-acetyl-2-methyl-chromen-4-on behandelte Probe:

Die Melanocyten zeigen eine sehr geringe Aktivität verbunden mit einem moderaten Melaningehalt in basalen Keratinocyten und geringem Gehalt an Melanin in suprabasalen Keratinocyten.

### Mit 5-Hydroxy-7-methoxy-2-methyl-chromen-4-on behandelte Probe

Die Melanocyten zeigen eine sehr geringe Aktivität, einhergehend mit einem moderaten Melaningehalt in basalen Keratinocyten und geringem Gehalt an Melanin in suprabasalen Keratinocyten.

### Chromatometrische Analyse:

Vergleichende Messungen am Starttag und nach 10 Tagen ergeben folgende Ergebnisse: Im Fall der unbehandelten Proben sind nach 10 Tagen nur geringfügige Veränderungen festzustellen. Mit Placebo behandelte Proben zeigen einen moderaten Anstieg des Hell/Dunkelparameters L an. Für die mit Verbindung la behandelten Proben lässt sich eine Abnahme des L-Parameters um 5,1% nachweisen, was die pro-pigmentierende Aktivität bestätigt (im Vergleich zur unbehandelten Kontrolle entspricht dies einer Steigerung von 43% der durch Melanin besetzten Fläche der basalen Schicht). Für Proben, die mit 5-Hydroxy-7-acetyl-2-methyl-chromen-4-on oder 5-hydroxy-7-methoxy-2-methyl-chromen-4-on behandelt wurden, läßt sich eine Zunahme des L-Parameters um 2,9% bzw. 5,1% nachweisen, was eher eine hautaufhellende Aktivität anzeigt.

### 2) Testserie mit UV-Bestrahlung:

| | | L | %L |
|---|---|---|---|
| Unbehandelte Probe | D0 | 65.87 | |
| | D10 | 64.56 | -2 |
| Mit Placebo behandelte Probe | D0 | 65.45 | |
| | D10 | 65.41 | -0.1 |
| Mit Verbindung la behandelte Probe | D0 | 64.07 | |
| | D10 | 61.52 | -4 |
| Mit 5-Hydroxy-7-acetyl-2-methyl-chromen-4-on behandelte Probe | D0 | 63.66 | |
| | D10 | 64.87 | 1.9 |
| Mit 5-hydroxy-7-methoxy-2-methyl-chromen-4-on behandelte Probe | D0 | 63.63 | |
| | D10 | 64.60 | 1.5 |

### Unbehandelte Proben:

Nach zehntägiger Behandlung zeigen die Melanocyten einen deutlichen Grad an dendritischer Verzweigung und sind eindeutig mit Melanin beladen. Es gibt gut nachweisbare Kontakte zu benachbarten Keratinocyten und ein moderates Maß an Melanintransfer. Melanin ist eindeutig nachweisbar in basalen Keratinocyten während in suprabasalen Keratinocyten nur ein geringer Melaningehalt nachzuweisen ist.

### Mit Placebo behandelte Proben:

Die Melanocyten zeigen ein hohes Maß an Aktivität. Der Melaningehalt in basalen Keratinocyten ist hoch, in suprabasalen niedrig.

### Mit Verbindung la behandelte Proben:

Die Melanocyten zeigen ein hohes Maß an Aktivität. Der Gehalt an Melanin in basalen Keratinocyten ist hoch, in suprabasalen moderat.

### Mit 5-Hydroxy-7-acetyl-2-methyl-chromen-4-on behandelte Probe:

Die Melanocyten zeigen geringe Aktivität verbunden mit einem moderaten Melaningehalt in basalen Keratinocyten und einem geringen Melaningehalt in suprabasalen Keratinocyten.

### Mit 5-hydroxy-7-methoxy-2-methyl-chromen-4-on behandelte Probe

Die Melanocyten zeigen eine sehr geringe Aktivität, die einhergeht mit einem moderaten Melaningehalt in basalen Keratinocyten und einem geringen Melaningahelt in suprabasalen Keratinocyten.

### Chromatometrische Analyse:

Vergleichende Messungen am Starttag und nach 10 Tagen ergeben folgende Ergebnisse: Die unbehandelten Proben zeigen nach 10 Tagen eine Abnahme des L-Parameters um 2.0%. Bei den mit Placebo behandelten Proben ist eine leichte Zunahme des L-Wertes um 2.2% festzustellen. Bei den mit Verbindung la behandelten Proben sinkt der Hell/Dunkelparameter L im Untersuchungszeitraum um 4.0%. Dies dokumentiert eine eindeutige pro-pigmentierende Aktivität der Verbindung la, insbesondere im Vergleich zum L-Wert der unbehandelten Proben. Für Proben, die mit 5-Hydroxy-7-acetyl-2-methyl-chromen-4-on oder 5-hydroxy-7-methoxy-2-methyl-chromen-4-on behandelt wurden, lässt sich eine Zunahme des L-Parameters nachweisen, was eher eine hautaufhellende Aktivität anzeigt.

### Beispiel 6: O/W-Formulierung

| **Bestandteile /Handelsname** | **Bezugsquelle** | **INCI** | **[Gew-%]** |
|---|---|---|---|
| **A** | | | |
| Marlipal 1618/11 | (1) | CETEARETH-11 | 3 |
| Lanette O | (2) | CETEARYLALCOHOL | 7 |
| Luvitol EHO | (3) | CETEARYLOCTANOATE | 5 |
| Tegosoft TN | (4) | C12-15 ALKYLBENZOATE | 2.5 |
| Miglyol 812 N | (1) | CAPRYLIC/CAPRIC TRIGLYCERIDE | 2.5 |
| Propyl-4-hydroxybenzoat | (5) | PROPYLPARABEN | 0.05 |
| Hexadecansäure 5-hydroxy-2-methyl-4-oxo-4H-chromen-7-yl ester **(Ia)** | | | 0.5 |
| | | | |

| **B** | | | |
|---|---|---|---|
| 1,2-Propandiol | (5) | PROPYLENE GLYCOL | 4 |
| Methyl-4-hydroxybenzoat | (5) | METHYLPARABEN | 0.15 |
| Wasser, demineralisiert | | AQUA (WATER) | ad 100 |
| | | | |
| | | | |
| | | | |
| Wasser, demineralisiert | | | 10 |
| **Gesamt** | | | **100.00** |

### Herstellungsverfahren:

Zunächst wird die Phase A auf 75°C und die Phase B auf 80 °C erwärmt. Danach wird Phase B unter Rühren langsam zu Phase A gegeben und solange gerührt, bis eine homogene Mischung entsteht.

### Bezugsquellen:

(1) Sasol Germany GmbH (2) Cognis GmbH (3) BASF AG (4) Degussa-Goldschmidt AG (5) Merck KGaA/Rona®

### Beispiel 7: O/W-Formulierung

| **Bestandteile /Handelsname** | **Bezugsquelle** | **INCI** | **[Gew-%]** |
|---|---|---|---|
| **A** | | | |
| Marlipal 1618/11 | (1) | CETEARETH-11 | 3 |
| Lanette O | (2) | CETEARYLALCOHOL | 7 |
| Luvitol EHO | (3) | CETEARYLOCTANOATE | 5 |
| Tegosoft TN | (4) | C12-15 ALKYLBENZOATE | 2.5 |
| Miglyol 812 N | (1) | CAPRYLIC/CAPRIC TRIGLYCERIDE | 2.5 |
| Propyl-4-hydroxybenzoat | (5) | PROPYLPARABEN | 0.05 |
| (E)-3,7-Dimethyl-octa-2,6-diensäure 5-hydroxy-2-methyl-4-oxo-4H-chromen-7-yl ester (Ib) | | | 0.1 |

| **B** | | | |
|---|---|---|---|
| 1,2-Propandiol | (5) | PROPYLENE GLYCOL | 4 |
| Methyl-4-hydroxybenzoat | (5) | METHYLPARABEN | 0.15 |
| Wasser, demineralisiert | | AQUA (WATER) | ad 100 |
| | | | |
| | | | |
| | | | |
| Wasser, demineralisiert | | | 10 |
| **Gesamt** | | | **100.00** |

### Herstellungsverfahren:

Zunächst wird die Phase A auf 75°C und die Phase B auf 80 °C erwärmt. Danach wird Phase B unter Rühren langsam zu Phase A gegeben und solange gerührt, bis eine homogene Mischung entsteht.

### Bezugsquellen:

(1) Sasol Germany GmbH (2) Cognis GmbH (3) BASF AG (4) Degussa-Goldschmidt AG (5) Merck KGaA/Rona®

### Beispiel 8: O/W-Formulierung

| **Bestandteile / Handelsname** | **Bezugsquelle** | **INCI** | **[Gew-%]** |
|---|---|---|---|
| **A** | | | |
| Tego Care 150 | (1) | GLYCERYL STEARATE, STEARETH-25, CETETH-20, STEARYL ALCOHOL | 8 |
| Lanette O | (2) | CETEARYL ALCOHOL | 1.5 |
| Luvitol EHO | (3) | CETEARYL OCTANOATE | 5 |
| Miglyol 812 N | (4) | CAPRYLIC/CAPRIC TRIGLYCERIDE | 5 |
| Paraffin liquid | (5) | PARAFFINUM LIQUIDUM (MINERAL OIL) | 3 |
| AbilWax 2434 | (1) | STEAROXY DIMETHICONE | 1.6 |
| Dow Corning 200 Fluid (350 cs) | (6) | DIMETHICONE | 0.5 |
| Propyl-4-hydroxybenzoat | (5) | PROPYLLPARABEN | 0.05 |
| | | | |

| **B** | | | |
|---|---|---|---|
| 1,2-Propandiol | (5) | PROPYLENE GLYCOL | 3 |
| Methyl-4-hydroxybenzoat | (5) | METHYLPARABEN | 0.15 |
| Wasser, entmineralisiert | | AQUA (WATER) | ad 100 |

| **C** | | | |
|---|---|---|---|
| | | | |
| Probiol L 05018 (Leere Liposomen) | (7) | AQUA, ALCOHOL DENAT, LECITHIN, GLYCERINE, DISODIUM PHOSPHATE | 5 |
| Wasser, entmineralisiert | | AQUA (WATER) | 10.00 |
| (E)-3-(4-Hydroxy-3-methoxyphenyl)-acrylsäure 5-hydroxy-2-methyl-4-oxo-4H-chromen-7-yl ester (Ic) | | | 0.2 |
| | | | |
| **Gesamt** | | | **100.00** |

### Herstellungsverfahren:

Zunächst werden die Phasen A und B auf 80 °C erwärmt. Danach wird Phase B unter Rühren langsam zu Phase A gegeben und homogenisiert. Dann wird abgekühlt und die Phase C bei 40°C zugegeben.

### Bezugsquellen:

(1) Degussa-Goldschmidt AG, (2) Cognis GmbH, (3) BASF AG, (4) Sasol Germany GmbH, (5) Merck KGaA/Rona®, (6) Dow Corning, (7) Kuhs GmbH & Co. KG

### Beispiel 9: W/O-Formulierung

| **Bestandteile / Handelsname** | **Bezugsquelle** | **INCI** | **[Gew-%]** |
|---|---|---|---|
| **A** | | | |
| Dow Corning 3225 C | (1) | CYCLOMETHICONE, DIMETHICONE COPOLYOL | 23.6 |
| Propyl-4-hydroxybenzoat | (2) | PROPYLPARABEN | 0.05 |
| (E)-3,7-Dimethyl-octa-2,6-diensäure 5-hydroxy-2-methyl-4-oxo-4H-chromen-7-yl ester (Ib) | | | 0.1 |

| **B** | | | |
|---|---|---|---|
| | | | |
| Methyl-4-hydroxybenzoat | (2) | METHYLPARABEN | 0.15 |
| 1,2-Propandiol | (2) | PROPYLENE GLYCOL | 35.9 |
| Wasser, entmineralisiert | | AQUA (WATER) | ad 100 |
| | | | |
| **Gesamt** | | | **100.00** |

### Herstellungsverfahren:

Zunächst wird die Phase B aufgelöst und dann wird sie zu Phase A gegeben. Der pH-Wert wird mit Natronlauge bzw. Citronensäure auf den Wert pH = 6.0 eingestellt.

### Bezugsquellen:

(1) Dow Corning (2) Merck KGaA/Rona®

### Beispiel 10: O/W Anti-aging Creme mit UV A/B Schutz

| **Bestandteile / Handelsname** | **Bezugsquelle** | **INCI** | **[Gew-%]** |
|---|---|---|---|
| **A** | | | |
| Eusolex^{®} 2292 | (1) | ETHYLHEXYL METHOXYCINNAMATE, BHT | 3 |
| Eusolex^{®} 4360 | (1) | BENZOPHENONE-3 | 0.5 |
| Tego Care 150 | (2) | GLYCERYL STEARATE, STEARETH-25, CETETH-20, STEARYL ALCOHOL | 8 |
| Lanette O | (3) | CETEARYL ALCOHOL | 1.5 |
| Luvitol EHO | (4) | CETEARYL OCTANOATE | 5 |
| Myglyol 812 N | (5) | CAPRYLIC/CAPRIC TRIGLYCERIDE | 5 |
| Paraffin liquid | (1) | PARAFFINUM LIQUIDUM (MINERAL OIL) | 3 |
| Abil-Wax 2434 | (2) | STEAROXY DIMETHICONE | 1.6 |
| Dow Corning 200 Fluid (350 es) | (6) | DIMETHICONE | 0.5 |
| Propyl-4-hydroxybenzoat | (1) | PROPYLPARABEN | 0.05 |
| Hexadecansäure 5-hydroxy-2-methyl-4-oxo-4H-chromen-7-yl ester (**Ia**) | | | 1 |
| | | | |

| **B** | | | |
|---|---|---|---|
| 1,2-Propandiol | (1) | PROPYLENE GLYCOL | 3 |
| Methyl-4-hydroxybenzoat Natriumsalz | (1) | SODIUM METHYLPARABEN | 0.17 |
| Wasser, demineralisiert | | AQUA (WATER) | ad 100 |
| | | | |
| **Gesamt** | | | **100.00** |

### Herstellungsverfahren:

Zunächst werden die Phasen A und B getrennt gemischt und auf 80°C erwärmt. Danach wird Phase B langsam unter Rühren zu Phase A gegeben. Es wird homogenisiert auf Raumtemperatur abgekühlt.

Bezugsquellen: (1) Merck KGaA/Rona®, (2) Degussa-Goldschmidt AG, (3) Cognis GmbH, (4) BASF AG, (5) Sasol Germany GmbH, (6)

## Patentansprüche

1. Verbindungen der Formel I 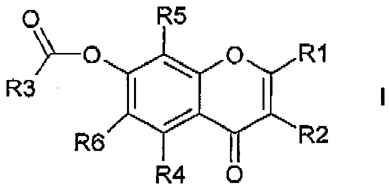 wobei
R1 und R2 unabhängig voneinander stehen für
-H,
-OH,
-geradkettiges oder verzweigtes O-(C₁- bis C₆-Alkyl) oder
-geradkettiges oder verzweigtes C₁- bis C₆-Alkyl,
wobei R1 und/oder R2 durch eine oder mehrere OH-Gruppen substituiert sein können und/oder wobei ein oder mehrere nicht benachbarte CH₂-Gruppen durch O ersetzt sein können;
R3 steht für
-geradkettige oder verzweigte C₆- bis C₂₀-Alkylgruppe,
-geradkettige oder verzweigte C₂- bis C₂₀-Alkenylgruppe,
-geradkettige oder verzweigte C₂- bis C₂₀-Alkinylgruppe,
-eine Cycloalkyl- oder Cycloalkenylgruppe mit 3 bis 6 C-Atomen, oder
-einen Rest der Formel III 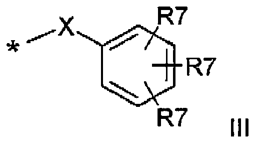 worin X für ein geradkettiges oder verzweigtes C₁- bis C₆-Alkylen oder geradkettiges oder verzweigtes C₂- bis C₆-Alkenylen steht und die Reste R7 unabhängig voneinander ausgewählt sind aus H, OH, geradkettiges oder verzweigtes C₁- bis C₆-Alkyl oder geradkettiges oder verzweigtes O-(C₁-bis C₆-Alkyl),
R4 für H, OH oder geradkettiges oder verzweigtes O-(C₁- bis C₂₀-Alkyl) steht,
R5 und R6 unabhängig voneinander stehen für
-H,
-OH,
-geradkettige oder verzweigte C₁- bis C₂₀-Alkylgruppe,
-geradkettige oder verzweigte C₂- bis C₂₀-Alkenylgruppe,
-geradkettige oder verzweigte C₁- bis C₂₀-Hydroxyalkylgruppe, wobei die Hydroxygruppe an ein primäres oder sekundäres Kohlenstoffatom der Kette gebunden sein kann und/oder ein oder mehrere nicht benachbarte CH₂-Gruppen durch O ersetzt sein können, oder
-geradkettige oder verzweigte O-(C₁- bis C₂₀-Alkyl)-Gruppe.

2. Verbindungen nach Anspruch 1, **dadurch gekennzeichnet, dass** R1 für H, OH, OCH₃, CH₃, CH₂O oder CH₂OCH₃ steht.

3. Verbindungen nach Anspruch 1 oder 2, **dadurch gekennzeichnet, dass** R2 für H, OH, OCH₃, CH₃, CH₂OH oder CH₂OCH₃ steht.

4. Verbindungen nach einem oder mehreren der Ansprüche 1 bis 3, **dadurch gekennzeichnet, dass** R3 für
- geradkettige oder verzweigte C₆- bis C₂₀-Alkylgruppe,
- geradkettige oder verzweigte C₂- bis C₂₀-Alkenylgruppe, oder
- einen Rest der Formel III 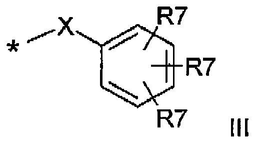 worin X für ein geradkettiges oder verzweigtes C₁- bis C₆-Alkylen oder geradkettiges oder verzweigtes C₂- bis Ce-Alkenylen steht und die Reste R7 unabhängig voneinander ausgewählt sind aus H, OH, geradkettiges oder verzweigtes C₁- bis C₆-Alkyl oder geradkettiges oder verzweigtes O-(C₁-bis C₆-Alkyl).

5. Verbindungen nach einem oder mehreren der Ansprüche 1 bis 4, **dadurch gekennzeichnet, dass** R4 für H, OH oder eine geradkettige oder verzweigte C₁- bis C₆-Alkylgruppe steht.

6. Verbindungen nach einem oder mehreren der Ansprüche 1 bis 5, **dadurch gekennzeichnet, dass** R5 und R6 unabhängig voneinander für H oder OH stehen.

7. Verbindungen nach einem oder mehreren der Ansprüche 1 bis 6, **dadurch gekennzeichnet, dass** die Verbindung der Formel I für eine Verbindung der Formel II steht 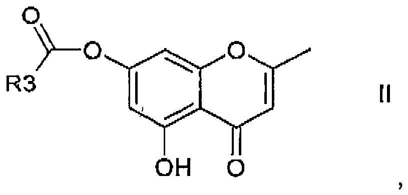 worin R3 wie in Anspruch 1 oder Anspruch 4 definiert ist.

8. Verbindungen nach einem oder mehreren der Ansprüche 1 bis 7, **dadurch gekennzeichnet, dass** die Verbindung der Formel I ausgewählt ist aus den Verbindungen der Formel la bis Id

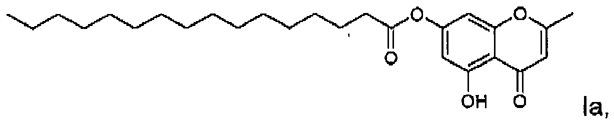

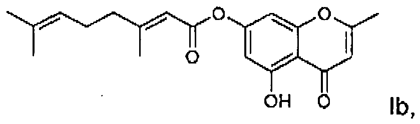

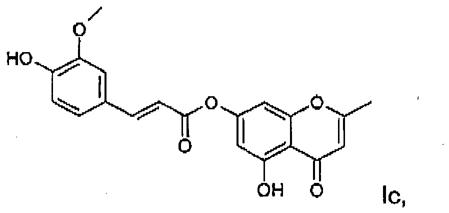

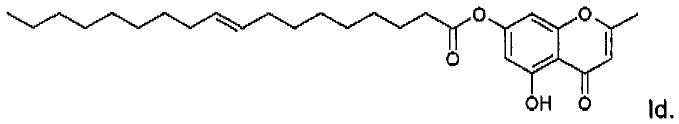

9. Verfahren zur Herstellung einer Verbindung der Formel I nach einem oder mehreren der Ansprüche 1 bis 8, **dadurch gekennzeichnet, dass** ein o-Hydroxyacetophenon der Formel IV 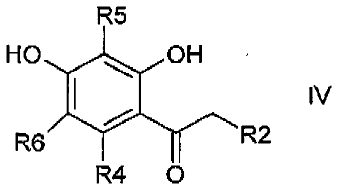 wobei die Substituenten R2 bis R6 wie in den Ansprüchen 1 bis 8 definiert sind, mit Acetylchlorid unter basischen Bedingungen cyclisiert wird und das Produkt anschließend in einer Veresterungsreaktion mit einer Verbindung der Formel R3-COCl umgesetzt wird.

10. Verbindung der Formel I nach einem oder mehreren der Ansprüche 1 bis 8 zur Verwendung als Selbstbräunungssubstanz.

11. Verbindung der Formel I nach einem oder mehreren der Ansprüche 1 bis 8 zur Verwendung zur Steigerung der Melaninsynthese, Verbesserung des Melanintransports und/oder Verbesserung der Verteilung von Melanin in suprabasalen Schichten.

12. Zubereitung enthaltend mindestens eine Verbindung der Formel I nach einem oder mehreren der Ansprüche 1 bis 8.

13. Zubereitung nach Anspruch 12, **dadurch gekennzeichnet, dass** sie eine oder mehrere Verbindungen der Formel I in einer Menge von 0,01 bis 10 Gew.-% enthält.

14. Zubereitung nach Anspruch 12 oder 13, **dadurch gekennzeichnet, dass** mindestens eine weitere Selbstbräunungssubstanz enthalten ist.

15. Verfahren zur Herstellung einer Zubereitung nach einem oder mehreren der Ansprüche 12 bis 14, **dadurch gekennzeichnet, dass** mindestens eine Verbindung der Formel I mit einem für topische Anwendungen geeigneten Träger vermischt wird.

## Claims

1. Compounds of the formula I 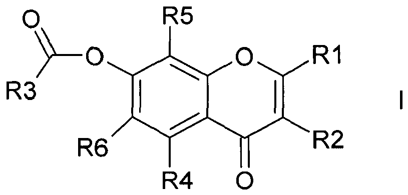 where
R1 and R2, independently of one another, stand for
-H,
-OH,
-straight-chain or branched O-(C₁- to C₆-alkyl) or
-straight-chain or branched C₁- to C₆-alkyl,
where R1 and/or R2 may be substituted by one or more OH groups and/or where one or more non-adjacent CH₂ groups may be replaced by O,
R3 stands for
-straight-chain or branched C₆- to C₂₀-alkyl group,
-straight-chain or branched C₂- to C₂₀-alkenyl group,
-straight-chain or branched C₂- to C₂₀-alkynyl group,
-a cycloalkyl or cycloalkenyl group having 3 to 6 C atoms, or
-a radical of the formula III 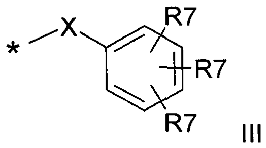 in which X stands for a straight-chain or branched C₁- to C₆-alkylene or straight-chain or branched C₂- to C₆-alkenylene and the radicals R7 are selected, independently of one another, from H, OH, straight-chain or branched C₁- to C₆-alkyl or straight-chain or branched O-(C₁- to C₆-alkyl),
R4 stands for H, OH or straight-chain or branched O-(C₁- to C₂₀-alkyl), R5 and R6, independently of one another, stand for
-H,
-OH,
-straight-chain or branched C₁- to C₂₀-alkyl group,
-straight-chain or branched C₂- to C₂₀-alkenyl group,
-straight-chain or branched C₁- to C₂₀-hydroxyalkyl group, where the hydroxyl group may be bonded to a primary or secondary carbon atom of the chain and/or one or more non-adjacent CH₂ groups may be replaced by O, or
-straight-chain or branched O-(C₁- to C₂₀-alkyl) group.

2. Compounds according to Claim 1, **characterised in that** R1 stands for H, OH, OCH₃, CH₃, CH₂OH or CH₂OCH₃.

3. Compounds according to Claim 1 or 2, **characterised in that** R2 stands for H, OH, OCH₃, CH₃, CH₂OH or CH₂OCH₃.

4. Compounds according to one or more of Claims 1 to 3, **characterised in that** R3 stands for
- straight-chain or branched C₆- to C₂₀-alkyl group,
- straight-chain or branched C₂- to C₂₀-alkenyl group, or
- a radical of the formula III 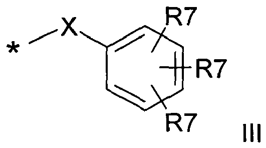 in which X stands for a straight-chain or branched C₁- to C₆-alkylene or straight-chain or branched C₂- to C₆-alkenylene and the radicals R7 are selected, independently of one another, from H, OH, straight-chain or branched C₁- to C₆-alkyl or straight-chain or branched O-(C₁- to C₆-alkyl).

5. Compounds according to one or more of Claims 1 to 4, **characterised in that** R4 stands for H, OH or a straight-chain or branched C₁- to C₆-alkyl group.

6. Compounds according to one or more of Claims 1 to 5, **characterised in that** R5 and R6, independently of one another, stand for H or OH.

7. Compounds according to one or more of Claims 1 to 6, **characterised in that** the compound of the formula I stands for a compound of the formula II 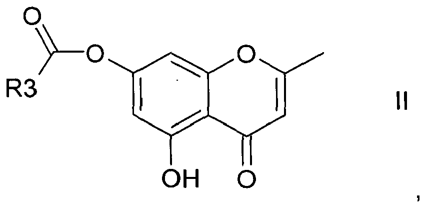 in which R3 is as defined in Claim 1 or Claim 4.

8. Compounds according to one or more of Claims 1 to 7, **characterised in that** the compound of the formula I is selected from the compounds of the formulae Ia to Id

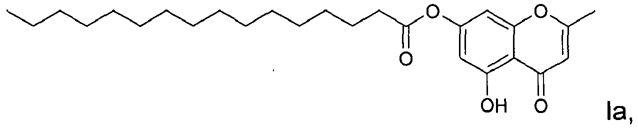

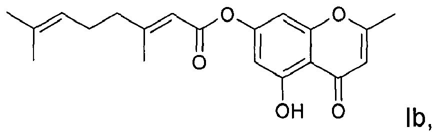

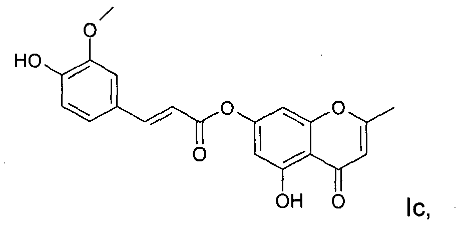

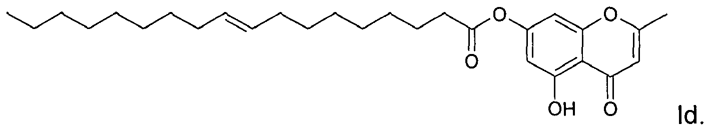

9. Process for the preparation of a compound of the formula I according to one or more of Claims 1 to 8, **characterised in that** an o-hydroxyacetophenone of the formula IV 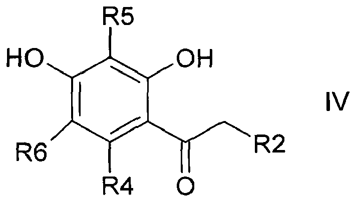 where the substituents R2 to R6 are as defined in Claims 1 to 8, is cyclised using acetyl chloride under basic conditions, and the product is subsequently reacted with a compound of the formula R3-COCl in an esterification reaction.

10. Compound of the formula I according to one or more of Claims 1 to 8 for use as self-tanning substance.

11. Compound of the formula I according to one or more of Claims 1 to 8 for use for increasing melanin synthesis, improving melanin transport and/or improving the distribution of melanin in suprabasal layers.

12. Preparation comprising at least one compound of the formula I according to one or more of Claims 1 to 8.

13. Preparation according to Claim 12, **characterised in that** it comprises one or more compounds of the formula I in an amount of 0.01 to 10% by weight.

14. Preparation according to Claim 12 or 13, **characterised in that** at least one further self-tanning substance is present.

15. Process for the preparation of a preparation according to one or more of Claims 12 to 14, **characterised in that** at least one compound of the formula I is mixed with a vehicle which is suitable for topical applications.

## Revendications

1. Composés de formule I 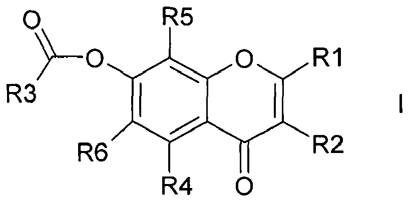 dans laquelle
R1 et R2, indépendamment l'un de l'autre, représentent
-H,
-OH,
- O-(C₁- à C₆-alkyl) à chaîne linéaire ou ramifiée ou
- C₁- à C₆-alkyle à chaîne linéaire ou ramifiée,
où R1 et/ou R2 peuvent être substitués par un ou plusieurs groupements OH
et/ou où un ou plusieurs groupements CH₂ non adjacents peuvent être remplacés par O,
R3 représente
- un groupement C₆- à C₂₀-alkyle à chaîne linéaire ou ramifiée,
- un groupement C₂- à C₂₀-alcényle à chaîne linéaire ou ramifiée,
- un groupement C₂- à C₂₀-alcynyle à chaîne linéaire ou ramifiée,
- un groupement cycloalkyle ou cycloalcényle ayant de 3 à 6 atomes de C, ou
- un radical de formule III 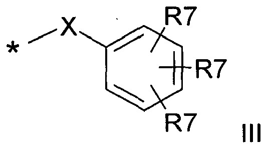 dans laquelle X représente C₁- à C₆-alkylène à chaîne linéaire ou ramifiée ou C₂- à C₆-alcénylène à chaîne linéaire ou ramifiée et les radicaux R7 sont choisis, indépendamment les uns des autres, parmi H, OH, C₁- à C₆-alkyle à chaîne linéaire ou ramifiée ou O-(C₁- à C₆-alkyl) à chaîne linéaire ou ramifiée,
R4 représente H, OH ou O-(C₁- à C₂₀-alkyl) à chaîne linéaire ou ramifiée,
R5 et R6, indépendamment l'un de l'autre, représentent
-H,
-OH,
- un groupement C₁- à C₂₀-alkyle à chaîne linéaire ou ramifiée,
- un groupement C₂- à C₂₀-alcényle à chaîne linéaire ou ramifiée,
- un groupement C₁- à C₂₀-hydroxyalkyle à chaîne linéaire ou ramifiée,
où le groupement hydroxyle peut être lié à un atome de carbone primaire ou secondaire de la chaîne et/ou un ou plusieurs groupements CH₂ non adjacents peuvent être remplacés par O, ou
- un groupement O-(C₁- à C₂₀-alkyl) à chaîne linéaire ou ramifiée.

2. Composés selon la revendication 1, **caractérisés en ce que** R1 représente H, OH, OCH₃, CH₃, CH₂OH ou CH₂OCH₃.

3. Composés selon la revendication 1 ou 2, **caractérisés en ce que** R2 représente H, OH, OCH₃, CH₃, CH₂OH ou CH₂OCH₃.

4. Composés selon l'une ou plusieurs parmi les revendications 1 à 3, **caractérisés en ce que** R3 représente
- un groupement C₆- à C₂₀-alkyle à chaîne linéaire ou ramifiée,
- un groupement C₂- à C₂₀-alcényle à chaîne linéaire ou ramifiée, ou
- un radical de formule III 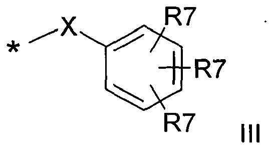 dans laquelle X représente C₁- à C₆-alkylène à chaîne linéaire ou ramifiée ou C₂- à C₆-alcénylène à chaîne linéaire ou ramifiée et les radicaux R7 sont choisis, indépendamment les uns des autres, parmi H, OH, C₁- à C₆-alkyle à chaîne linéaire ou ramifiée ou O-(C₁- à C₆-alkyl) à chaîne linéaire ou ramifiée.

5. Composés selon l'une ou plusieurs parmi les revendications 1 à 4, **caractérisés en ce que** R4 représente H, OH ou un groupement C₁- à C₆-alkyle à chaîne linéaire ou ramifiée.

6. Composés selon l'une ou plusieurs parmi les revendications 1 à 5, **caractérisés en ce que** R5 et R6, indépendamment l'un de l'autre, représentent H ou OH.

7. Composés selon l'une ou plusieurs parmi les revendications 1 à 6, **caractérisés en ce que** le composé de formule I représente un composé de formule II 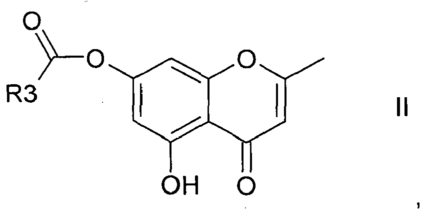 dans laquelle R3 est tel que défini selon la revendication 1 ou la revendication 4.

8. Composés selon l'une ou plusieurs parmi les revendications 1 à 7, **caractérisés en ce que** le composé de formule I est choisi parmi les composés de formules la à Id

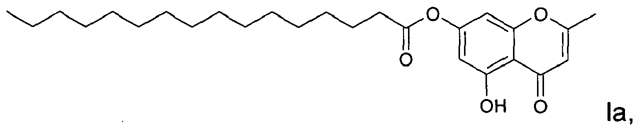

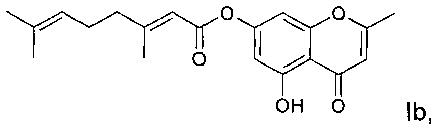

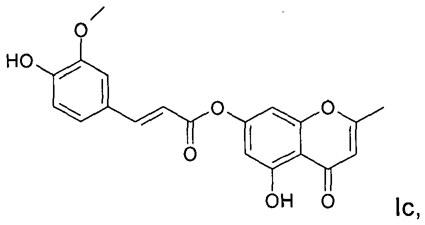

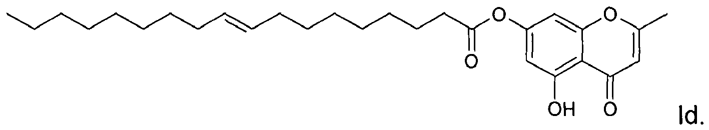

9. Procédé de préparation d'un composé de formule I selon l'une ou plusieurs parmi les revendications 1 à 8, **caractérisé en ce qu'**une o-hydroxyacétophénone de formule IV 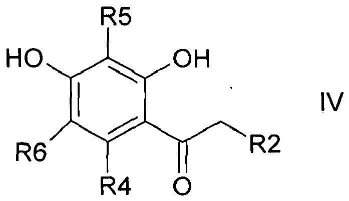 dans laquelle les substituants R2 à R6 sont tels que définis selon les revendications 1 à 8, est cyclisée en utilisant du chlorure d'acétyle dans des conditions basiques, et le produit est ensuite réagi avec un composé de formule R3-COCI dans une réaction d'estérification.

10. Composé de formule I selon l'une ou plusieurs parmi les revendications 1 à 8, pour une utilisation comme substance auto-bronzante.

11. Composé de formule I selon l'une ou plusieurs parmi les revendications 1 à 8, pour une utilisation dans l'augmentation de la synthèse de la mélanine, l'amélioration du transport de la mélanine et/ou l'amélioration de la distribution de la mélanine dans les couches suprabasales.

12. Préparation comprenant au moins un composé de formule I selon l'une ou plusieurs parmi les revendications 1 à 8.

13. Préparation selon la revendication 12, **caractérisée en ce qu'**elle comprend un ou plusieurs composés de formule I selon une quantité allant de 0,01 à 10% en poids.

14. Préparation selon la revendication 12 ou 13, **caractérisée en ce qu'**au moins une substance auto-bronzante supplémentaire est présente.

15. Procédé de préparation d'une préparation selon l'une ou plusieurs parmi les revendications 12 à 14, **caractérisé en ce qu'**au moins un composé de formule I est mélangé avec un véhicule qui est convenable pour des applications topiques.
